# EUROPÄISCHE PATENTANMELDUNG

(11) **EP 4 685 132 A1**
(43) Veröffentlichungstag der Anmeldung: **28.01.2026**
(21) Anmeldenummer: 24191101.5
(22) Anmeldetag: 26.07.2024
(51) Int. Cl.: C07C 209/68, C07C 211/36, B01J 8/00

(54) **VERFAHREN ZUR KONTINUIERLICHEN, KATALYTISCHEN HYDRIERUNG VON MDA**

(71) Anmelder: Evonik Operations GmbH, 45128 Essen (DE)
(72) Erfinder: KUHLMANN, Hanns, 45549 Sprockhövel (DE); RIX, Armin Matthias, 45770 Marl (DE); PAUL, Niklas, 45770 Marl (DE); WESSNER, Lea, 44139 Dortmund (DE); VARGAS GÓMEZ, Maria, 45721 Haltern am See (DE); WINKLER, Tobias, 48249 Dülmen (DE); BOECK, Florian, 58455 Witten (DE); SUDHOFF, Daniel, 59192 Bergkamen (DE)
(74) Vertreter: Evonik Patent Association

(57) **Zusammenfassung**

Anlage zur kontinuierlichen, katalytischen Hydrierung von Methylendianilin (MDA; Edukt1) mit einem Wasserstoffgeber (Edukt2), insb. einem gasförmigen Wasserstoffgeber, vorzugsweises Wasserstoff (H2), umfassend eine Konditionierungseinheit für die Edukte, eine Reaktoreinheit zur Synthese von PACM und eine Trenneinheit, wobei
- die Konditionierungseinheit mind. eine Teillänge) der (Zu-)Leitungen für Edukte1, Edukt2 und mind. ein Lösungsmittel, mind. einen Wärmetauscher in mind. einer (Zu-)Leitung, mind. einen Mischer zur Mischung der Edukte und/oder mind. eines Edukts mit mind. einem Lösungsmittel umfasst;
- die Reaktoreinheit mind. einen Festbettreaktor als Hauptreaktor mit einer immobilen Katalysatorpackung umfasst, wobei der mind. eine (erste) Hauptreaktor
- einen ersten Strömungsweg für das Stoffgemisch
über die immobile Katalysatorpackung und
- einen weiteren hiervon getrennten, geschlossenen Strömungsweg für ein Wärmetauschmittel außerhalb der Katalysatorpackung umfasst, und
wobei in den Medienumlaufein Wärmetauscher eingebunden ist;
- die Trenneinheit mind.
- eine erste Trennstufe zur (im Wesentlichen) Abtrennung des Lösungsmittels und
- eine zweite Trennstufe zur (im Wesentlichen) Abtrennung vom Edukt und Nebenprodukten vom Produkt PACM umfasst, wobei

i) ein Sammelkreislauf für einen geschlossenen ersten Medienumlauf umfasst ist, in den als mind. eine Wärmequelle der Wärmetauscher des Reaktors und/oder mind. ein Wärmetauscher der Trenneinheit sowie ein Verdampfer eingebunden sind,
ii) ein Zwischenkreislauf für einen geschlossenen zweiten Medienumlauf umfasst ist, in den der erste Verdampfer, mind. ein Verdichter und ein Verteilerkessel eingebunden sind, und wobei
iii) mind. ein Verteilerkreislauf für einen geschlossenen dritten Medienumlauf umfasst ist, in den als Wärmesenke mind. ein Wärmetauscher der Reaktoreinheit, mind. ein Wärmetauscher der der Konditionierungseinheit und/oder mind. ein Wärmetauscher der Trenneinheit wärmetauschen eingebunden ist.

## Beschreibung

Die Erfindung betrifft eine Anlage und ein Verfahren zur kontinuierlichen, katalytischen Hydrierung von MDA, insb. zur Herstellung von Methylenbis(cyclohexylamin), wie insb. 4,4'-Diaminodicyclohexylmethan (PACM).

Verfahren zur Hydrierung organischer Verbindungen, insbesondere zur Hydrierung von aromatischen Verbindungen zu den entsprechenden Cyclohexan-Derivaten sind aus dem Stand der Technik bereits bekannt.

Methylenbis(cyclohexylamin) ist ein bei Standardbedingungen (SATP) festes oder flüssig vorliegendes cycloaliphatisches Amin, das üblicherweise über die Flüssigphasen-Hydrierung von MDA hergestellt wird. Das Akronym MDA wurde historisch als Abkürzung für das bei der Umsetzung von Anilin und Formaldehyd gebildete, vor allem "Methylendianilin" (Diaminodiphenylmethan) umfassende Produktgemisch eingeführt und wird weiterhin zur Bezeichnung des mittlerweile großtechnisch erzeugten Verfahrensproduktes verwendet. Das Produkt der Hydrierung, das vor allem Methylenbis(cyclohexylamin) umfasst, wird deswegen oft auch als H12MDA bezeichnet.

MDA ist aufgrund seines Herstellungsprozesses üblicherweise ein Gemisch aus verschiedenen Diaminodiphenylmethanen. Vor allem besteht es aus 4,4`-Diaminodiphenylmethan. Es können jedoch auch 2,4`- und 2,2'-lsomere vorliegen. MDA kann weiterhin bei der Umsetzung von Anilin und Formaldehyd gebildete Reaktionsprodukte mit drei oder mehr aromatischen Ringen, insbesondere solche mit drei oder mehr Phenylringen, enthalten. Diese Reaktionsprodukte mit drei oder mehr aromatischen Ringen werden auch als Mehrkernverbindungen bezeichnet.

Bei dem im Handel erhältlichen Methylenbis(cyclohexylamin) handelt es sich aufgrund des hohen Anteils an 4,4`-Diaminodiphenylmethan im eingesetzten MDA größtenteils um 4,4'-Diaminodicyclohexylmethan bzw. Bis(para-Aminocyclohexyl)methan. Aufgrund der potentiellen Anwesenheit der entsprechenden 2,4'- und 2,2'-Diaminophenylmethan-lsomere im MDA kann in Methylenbis(cyclohexylamin) auch 2,4`-Diaminodicyclohexylmethan und 2,2'-Diaminodicyclohexylmethan vorliegen. Weiterhin kann hydriertes MDA neben Methylenbis(cyclohexylamin) weiterhin noch (ggf. partiell) hydrierte Mehrkernverbindungen enthalten.

US 5,578,546 A offenbart, dass 1947 erstmalig ein Verfahren zur Herstellung von Methylenbis(cyclohexylamin) beschrieben und 1965 in den technischen Maßstab überführt wurde. Die Hydrierung von MDA ist stark exotherm. So gibt WO 2010/069484 A1 eine Reaktionsenthalpie von -1600 kJ/mol an.

In Folge der Hydrierung werden je nach Verfahren verschiedene Diastereoisomere gebildet. Dabei kann das von 4,4`-Diaminodiphenylmethan abgeleitete Produkt 4,4'-Diaminodicyclohexylmethan (PACM) als trans/trans-, cis/cis- und cis/trans-Isomer vorliegen und ist deswegen in der Regel ein Gemisch dieser Isomere mit unterschiedlichen Anteilen. Mit steigendem trans/trans-Gehalt steigt dabei der Schmelzpunkt der Verbindung. Deshalb unterscheiden sich die Einsatzgebiete je nach Isomerengehalt stark: Während Methylenbis(cyclohexylamin)-Qualitäten mit niedrigem trans/trans - Gehalt (z.B. 10-30 Gew.-%) im Bereich der Amin- und Isocyanat-Vernetzer, insbesondere im Bereich von Zwei-Komponenten-Harzen Einsatz finden, finden Qualitäten mit hohem trans/trans - Gehalt (z.B. ≥ 48 Gew.-%) vor allem Anwendung als Regulator in Polyamidverbindungen. Gerade die Herstellung von Produkten mit niedrigem trans/trans-Gehalt stellt eine Herausforderung dar, da das thermodynamische Gleichgewicht, wie in US 3,636,108 A beschrieben wird, im Bereich wesentlich höherer trans/trans-Anteile (bis zu 51,2 %) liegt. US 2,606,925 A zeigt zudem, dass das Gleichgewicht nachträglich durch längeres Temperieren in Richtung eines höheren Anteils an trans/trans-Isomer verschoben werden kann.

Die Zusammensetzung des Hydrierungsproduktes hängt auch von der Zusammensetzung des eingesetzten MDAs ab: Oft wird MDA in Qualitäten von MDA50 bis MDA100 eingesetzt, wobei die zwischen 50 und 100 liegende Zahl den Gehalt an Diaminodiphenylmethanen im MDA-Gemisch angibt. MDA50 ist dabei eine MDA-Qualität, die wie oben erläutert prozessbedingt etwa 50 Gew.-% Diaminodiphenylmethanen und 50 Gew.-% Mehrkernverbindungen enthält. Die einzelnen Mehrkernverbindungen können entsprechend der enthaltenen Aromatenanzahl als 3-Kern-Verbindungen, 4-Kern-Verbindungen, etc. bezeichnet werden. MDA50 ist dabei die am meisten produzierte Qualität und wird hauptsächlich zu Methylendicyclohexyldiisocyanat (MDI) weiterverarbeitet. MDA100 ist reines MDA bzw. Diaminodiphenylmethan ohne Mehrkernverbindungen. MDA85 oder MDA90 sind andere auf dem Markt verfügbare Qualitäten mittlerer Reinheit. Wenn in Patentschriften zum Herstellverfahren von Methylenbis(cyclohexylamin) auf die Reinheit der MDA-Qualität eingegangen wird, so ist meist von MOA 100 die Rede (z.B. CN 110204447 B). Dagegen stellt US 2005/261525 A1 bevorzugt auf die Hydrierung von MDA50 ab. Die dabei als Hochsieder anfallenden hydrierten, oligomeren Amine eignen sich als Vernetzer mit besonders niedrigen Dampfdrücken für eine Reihe von Spezialanwendungen, wie US 2004/162409 A1 zeigt.

Der Gehalt an (ggf. partiell) hydrierten Mehrkernverbindungen im Produkt nimmt in der Reihenfolge der Edukte MDA50, MDA85, MDA90, MDA100 ab, da der Gehalt an Mehrkernverbindungen von MDA50 zu MDA100 abnimmt.

Aus der WO 2009/153123 A1 ist ein kontinuierliches Verfahren und einen Reaktor zur Hydrierung organischer Verbindungen in einem mehrphasigen, mehrstufigen System in Gegenwart eines homogenen oder heterogenen Katalysators bekannt. Als Katalysatoren werden u.a. Edelmetalle, wie Platin, Palladium, Ruthenium und Rhodium oder andere Übergangsmetalle, wie Molybdän, Wolfram und Chrom vorgeschlagen. Hierbei können die heterogenen Katalysatoren auf Trägermaterialien angeordnet sein, wie bspw. Kohlenstoff, Aluminiumoxid, Siliciumdioxid, Zirkoniumdioxid, Zeolithe, Alumosilikate oder Mischungen dieser Trägermaterialien. Als Substrate werden in diesem Verfahren bevorzugt aromatische Verbindungen enthaltend Amino-Substituenten eingesetzt, beispielsweise MDA, Polymer-MDA, Anilin, 2,4-Diaminotoluol, 2,6-Diaminotoluol, o-Phenylendiamin, etc. Die heterogenen Katalysatoren werden in Suspension eingesetzt.

DE 19533718 A1 offenbart ein Verfahren zur Hydrierung von aromatischen Verbindungen, in denen mindestens eine Aminogruppe an einen aromatischen Kern gebunden ist. Dazu kann ein heterogener Katalysator enthaltend Ruthenium und gegebenenfalls mindestens ein Metall der I-, VII- oder VIII-Nebengruppe verwendet werden. Als Trägermaterial wird beispielsweise Aluminiumoxid, Siliciumdioxid, Titandioxid oder Zirkoniumdioxid, vorzugsweise Aluminiumdioxid oder Zirkoniumdioxid, eingesetzt. Als Beispiel angegeben wird lediglich ein Katalysator enthaltend Ruthenium auf dem Trägermaterial Aluminiumoxid, nicht jedoch Zirkoniumoxid.

EP 1337331 A1 offenbart ein Verfahren zur katalytischen Hydrierung von aromatischen oder heteroaromatischen Aminen, wobei als Aktivmetall Ruthenium fungiert und der Katalysator mindestens ein weiteres Metall der I-, VII-, oder VIII-Nebengruppe enthält und diese auf einem Trägermaterial aufgebracht sind. Hierbei werden als aromatische Verbindungen u.a. 4,4'-MDA und Isomeren hiervon eingesetzt. Auch die EP 0111238 A1 offenbart ein Verfahren zur katalytischen Hydrierung von 4,4'-MDA, dadurch gekennzeichnet, dass die Hydrierung in Gegenwart von geträgertem Ruthenium in Anwesenheit von Nitraten und Sulfaten der Alkalimetalle und Nitraten der Erdalkalimetalle erfolgt. Ein vergleichbares Verfahren wird in der EP 1366812 A1 offenbart, wobei als Trägermaterialien unter anderem Aluminiumoxid, Siliciumoxid, Titanoxid und Zirkoniumoxid genannt werden.

Weitere Verfahren zur Hydrierung organischer Verbindungen offenbaren WO 2011/003899 A1 und WO 2009/090179 A1. Hier werden Verfahren zur Hydrierung von aromatischen Aminen mit Wasserstoff in Gegenwart eines Ru-Katalysators, welcher, u.a. Zirkoniumoxid-Trägermaterial enthält, offenbart.

Schließlich ist aus der EP 2 883 863 B1 ein Verfahren und eine Anlage zur Hydrierung von 4,4'-Methylendianilin (MDA) und/oder Polymer-MDA mit Wasserstoff in Gegenwart eines Katalysators bekannt. Hierbei wird als Katalysator Ruthenium vorgeschlagen, welcher auf einem Zirkoniumoxid-Trägermaterial aufgebracht ist. Bezüglich des Reaktors, verweist die EP 2 883 863 B1 auf den Reaktor bzw. das Reaktorkonzept der WO 2008/015135 A1. Aus dieser WO 2008/015135 A1 ist ein kontinuierliches Verfahren und eine Anlage zur Hydrierung von Diisononylphthalat zu 1,2-Cyclohexandicabonsäurediisononylester (DINCH) bekannt, wobei DINP als Gemisch in einem organischen Lösungsmittel, mit Wasserstoff bei einem Druck von bis zu 325 bar hydriert wird. Hierbei wird eine in Reihenschaltung von zwei Festbettreaktoren mit jeweils einer immobilen Festbettschüttung vorgeschlagen. Zur Ableitung der Reaktionswärme aus den beiden Reaktoren wird vorgeschlagen, einen Teilstrom des Stoffgemisches nach dem zweiten Festbettreaktor zu rezirkulieren und diesen hierbei zu kühlen. Ein vergleichbares Reaktorkonzept von in Reihe geschalteten Festbettreaktoren zur Hydrierung von ist auch aus der EP 1 566 372 B1 bekannt.

Nachteilig an dem Konzept ist, dass die Kreislaufführung eines Produktteilstroms zu einer erhöhten Bildung von unerwünschten Nebenprodukten führen kann und die Anlagenleistung senkt, wobei erhöhte Energiekosten für die Rückführung und Kühlung des Recyclingstroms erzeugt werden.

Wie ausgeführt, ist der Bedarf an bspw. PACM mit unterschiedlichen Anteilen der jeweiligen Isomeren abhängig vom dem Einsatzzweck bzw. den nachfolgenden Produkten. So sind beispielsweise PACM-Qualitäten mit niedrigem trans/trans-Gehalt von 10 bis 30 Gew.% im Bereich der Amin- und Isocyanat-Vernetzer bevorzugt, insbesondere im Bereich der Formulierung von 2-Komponenten-Harzen, wobei PACM-Qualitäten mit hohem trans/trans-Gehalt von über 48 Gew.% vor allem Anwendung als Regulator in Polyamidverbindungen finden. Die genannten Gew.% beziehen sich hierbei auf das PACM-Isomerengemisch als solches. Gerade die Herstellung von Produkten mit niedrigem trans/trans-Gehalt stellt eine verfahrenstechnische Herausforderung dar, da das thermodynamische Gleichgewicht, wie in US 3,636,108 A beschrieben wird, im Bereich wesentlich höherer trans/trans-Anteile von bis zu 51,2 Gew.% liegt. Weiterhin ist aus der US 2,606,925 A bekannt, dass das Gleichgewicht der PACM-Isomere durch längeres Temperieren nachträglich in Richtung zu einem höheren Anteil an trans/trans-Isomer verschoben wird.

Aufgabe der vorliegenden Erfindung ist es somit, eine verbesserte Anlage und einen verbesserten Prozess bereitzustellen, der hinsichtlich Produktumsatz und Energieeffizienz verbessert ist und insb. die Herstellung definierter Anteile der jeweiligen Isomere im Isomerengemisch ermöglicht.

Die Aufgabe wird erfindungsgemäß mit einer Anlage nach den Merkmalen des Anspruch 1 und einem Verfahren nach den Merkmalen des Anspruchs 14 gelöst.

Hierbei wird eine Anlage vorgesehen, zur kontinuierlichen, katalytischen Hydrierung von Methylendianilin (MDA; Edukt1) mit einem Wasserstoffgeber (Edukt2), insb. einem gasförmigen Wasserstoffgeber, vorzugsweises Wasserstoff (H2), umfassend eine Konditionierungseinheit (104) für die Edukte, eine Reaktoreinheit (102) und eine Trenneinheit (106), wobei
- die Konditionierungseinheit (Zu-)Leitungen für Edukte1, Edukt2 und mind. ein Lösungsmittel, mind. einen Wärmetauscher in mind. einer (Zu-)Leitung, mind. einen Mischer zur Mischung der Edukte und/oder mind. eines Edukts mit mind. einem Lösungsmittel umfasst;
- die Reaktoreinheit mind. einen Festbettreaktor als Hauptreaktor mit einer immobilen Katalysatorpackung umfasst, wobei der mind. eine Hauptreaktor
   - einen ersten Strömungsweg für das Stoffgemisch
      über die immobile Katalysatorpackung und

   - einen weiteren hiervon getrennten, geschlossenen Strömungsweg für ein Wärmetauschmedium außerhalb der Katalysatorpackung umfasst, und
      wobei in den Medienumlauf ein Wärmetauscher eingebunden ist;
- die Trenneinheit mind.
- eine erste Trennstufe zur Abtrennung des Lösungsmittels und
- eine zweite Trennstufe zur Abtrennung vom mind. einem Edukt und/oder mind. einem Nebenprodukt vom Produkt umfasst, wobei
   i) ein Sammelkreislauf für einen offenen oder einen geschlossenen ersten Medienumlauf umfasst ist, in den als Wärmequelle/n mind. der Wärmetauscher des Hauptreaktors und/oder mind. ein Wärmetauscher der Trenneinheit, sowie ein Verdampfer eingebunden sind,
   ii) ein Zwischenkreislauf für einen zweiten Medienumlauf umfasst ist, in den der erste Verdampfer, mind. ein Gebläse und ein Verteilerkessel eingebunden sind, wobei von der Druckseite des mind. einen Gebläses eine hinführende (Medien-)Leitung zum Verteilerkessel führt und ein (Sumpf-)Auslass des Verteilerkessels über eine rückführende Leitung mit einem Einlass des Verdampfers verbunden ist, und wobei
   iii) mind. ein Verteilerkreislauf umfasst ist, in den der Verteilerkessel eingebunden ist, wobei der Verteilerkreislauf mit mind. einem hinführenden Leitungsast an einen Kopf-/Dampfauslass des Verteilerkessels angeschlossen ist, wobei der Verteilerkreislauf mind. einen verteilenden Leitungsast und mind. einen rückführenden Leitungsast umfasst, wobei in den mind. einen verteilenden Leitungsast als Wärmesenke mind. ein Wärmetauscher der Konditionierungseinheit, der Trenneinheit, und/oder der Reaktionseinheit wärmetauschend eingebunden sind, und wobei der Zwischenkreislauf und der Verteilerkreislauf über den Verteilerkessel medienleitend miteinander verbunden sind.

Hierbei umfasst
- der Sammelkreislauf einen zum ersten Verdampfer hinführenden oder Energie sammelnden Leitungsast und einen rückführenden Leitungsast,
- der Zwischenkreislauf einen zum Verteilerkessel hinführenden Leitungsast, auch Dampfleitung genannt, sowie einen vom Verteilerkessel zum ersten Verdampfer rückführenden Leitungsast, auch Rückleitung genannt, und
- der Verteilerkreislauf mindestens einen hinführenden Leitungsast, auch Leitungsabschnitt genannt, vom Verteilerkessel zu dem mind. einen als Wärmesenke eingebundenen Wärmetauscher der Konditionierungseinheit, Trenneinheit und/oder Reaktoreinheit und weiterhin einen rückführenden Leitungsast von dem mind. einen als Wärmesenke wirkenden Wärmetauscher, insb. der Gruppe aus mind. zwei Wärmetauschern, hin zum Verteilerkessel.

Die Besonderheit dieser drei Kreisläufe ist, dass der Sammelkreislauf ein geschlossener Medienkreislauf oder ein offener Medienkreislauf ist. Hierbei meint im vorliegenden Zusammenhang "geschlossener (Medien-)Kreislauf", dass das Wärmetauschmedium, i.d.R. Wasser, in einem geschlossenen Kreislauf geführt wird. Der Wärmeaustausch vom Sammelkreislauf mit dem Zwischenkreislauf erfolgt in einem indirekten Wärmeübertrag über den Verdampfer. In einem geschlossenen Kreislauf kann das (Wärmetausch-)Medium nur in dem jeweiligen Kreislauf zirkulieren, insb. nicht in den benachbarten Kreislauf weitergeleitet werden, so dass der Energieaustausch mit dem jeweils gekoppelten Nachbarkreislauf über einen indirekten Wärmetransport zwischen den zirkulierenden (Kreislauf-)Medien erfolgt, ohne Austausch der jeweiligen (Kreislauf-)Medien.

Der Zwischenkreislauf ist hierbei gekoppelt mit dem Sammelkreislauf und dem mind. einen Verteilerkreislauf. Der Sammelkreislauf dient als Wärmequelle für den Zwischenkreislauf, der Zwischenkreislauf dient zum Heben des Temperaturniveaus und als Wärmequelle für den Verteilerkreislauf und die dort eingebundenen als Wärmesenke fungierenden Wärmetauscher, insb. die Wärmetauscher der Trenneinheit.

Im Unterschied hierzu meint im vorliegenden Zusammenhang "offener (Medien-)Kreislauf", dass das Wärmetauschmedium, i.d.R. Wasser, von einem Kreislauf mindestens teilweise bzw. mind. in einem Aggregatszustand in einen benachbarten Kreislauf geleitet bzw. von dort entnommen oder empfangen wird. Der Medienaustausch bei offenen (Medien-)Kreisläufen erfolgt insb. über Verteilerkessel.

Somit meint im vorliegenden Zusammenhang der Begriff "Verdampfer", dass ein indirekter Wärmeübergang erfolgt und kein stofflicher Austausch zwischen zufließenden Stoffen oder Medien erfolgt. Im Unterschied hierzu meint im vorliegenden Zusammenhang "Verteilerkessel", dass mind. Teilmengen aus zwei oder mehr (Teil-)Kreisläufen zufließenden Medien stofflich gemischt werden, d.h. zusammenfließen. Hierbei kann ein Verteilkessel zusätzlich ein Wärmetauschelement umfassen oder hieran angeschlossen sein, mittels welchem ein indirekter Wärmeübergang erfolgen kann.

Der Medienumlauf des Zwischenkreislaufs und des Verteilkreislaufs sind über den Verteilerkessel fluidisch gekoppelt, so dass dasselbe Wärmetauschmedium, in der Regel Wasser bzw. Wasserdampft, durch die beiden Kreisläufe strömt. Der Verteilerkessel bildet hierbei eine Art gemeinsamer Strömungs- oder Leitungsknoten für den Zwischenkreislauf und den Verteilerkreislauf.

Der "Verteilerkessel" soll weiterhin vorliegend nicht einschränkend verstanden werden und stellt einen beliebig geformten Kessel oder Raum dar, in dem eine Phasentrennung in Dampf- und Flüssigphase erfolgen kann, so dass die flüssige Phase getrennt zum Verdampfer zurückgefördert werden kann, bspw. aus dem Sumpf eines Kessels. So weist der Verteilerkessel einen Sumpfauslass auf an den sich eine Sumpfleitung anschließt und mind. eine (Sumpf-)Pumpe angeschlossen ist. Die (Sumpf-)Pumpe beschickt mind. die rückführende Leitung des Zwischenkreislaufs mit (Wärmetausch-)Medium hin zum Verdampfer, wo dieses erneut mind. teilweise verdampft wird.

Der von dem Trennkessel kommende flüssige Stoffstrom wird über eine Leitung zur ersten Trennkolonne innerhalb der ersten Trennstufe der Trenneinheit geleitet. Vorteilhafterweise kann bei einer Ausführungsform der Anlage vorgesehen sein, dass in der Leitung vom Trennkessel zur ersten Kolonne eine Druckregeleinheit zur Druckabsenkung vorgesehen ist. Hierdurch kann die Reaktoreinheit auf einem ersten, hohen Druckniveau betrieben werden und die ersten Trennstufe der Trenneinheit auf einem zweiten, tieferen Druckniveau.

Die Anlage dient vorzugsweise zur kontinuierlichen, katalytischen Herstellung von Methylenbis(cyclohexylamin) als Produkt, insb. zur Produktion von 4,4'-Diaminodicyclohexylmethan (PACM), nach der Formel (I)

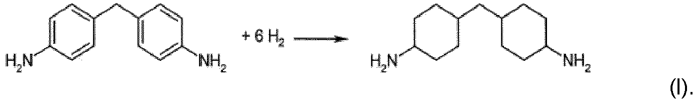

Mit dem separaten Nachreaktor, insb. dem adiabatischen Nachreaktor, ist eine optimierte Steuerung des Prozesses und der Anlage möglich geworden, wodurch die Selektivität durch vom Hauptreaktor (Auslass) abweichende Eintrittstemperatur des Nachreaktors möglich wird. Typischerweise kann die Eintrittstemperatur des Nachreaktors bei der gleichen oder einer geringfügig niedrigeren Temperatur vorgesehen werden, die bis zu 30 °C unterhalb der Auslasstemperatur des Hauptreaktors liegen kann. Somit kann mind. zeitweise eine Temperaturerhöhung im (adiabatischen) Nachreaktor von 20 °C bis 40 °C, typischerweise von 20 °C bis 30 °C zugelassen werden, und so gezielt die gewünschte Produktqualität (Isomerenverhältnis) eingestellt werden . Auf diese Weise ist ein sehr niedriger und ein sehr genauer Anteil an trans/trans-Isomeren in dem Isomerengemisch einstellbar. Der Hauptreaktor kann auf einem möglichst niedrigen Temperaturniveau betrieben werden, so dass der trans/trans-Anteil des PACM im Stoffstrom (Auslass des Hauptreaktors) ca. 13 bis 20 Gew.% beträgt, wobei ca. 13 % bei einem neuen bzw. regenerierten Katalysator erreichbar sind und 20 Gew.% bei einem Katalysator nach Langzeitnutzung (kurz vor Austausch/Regeneration). Abhängig von der Phase, in der sich der Hauptreaktor befindet, kann es mind. zeitweise sinnvoll sein, wenn die Eintrittstemperatur des Nachreaktors um 5 bis 20 °C höher als die des Hauptreaktors ist.

Der mit Katalysator frisch befüllte oder regenerierte Hauptreaktor mit der dortigen stark exothermen Reaktion wird hierbei weitgehend isotherm betrieben, was aber nicht im idealen Sinne zu verstehen ist. Auch wenn der Hauptreaktor vorliegend als "isotherm/-isch" bezeichnet wird, wird dieser ideale Zustand bei industrieller Anwendung nur bedingt erreicht, so dass sich innerhalb des Hauptreaktors wegen unvollständigem Wärmeabtransport ein Temperaturgradient von ca. 5 bis 10 °C in radiale und auch in Strömungsrichtung ausbildet.

Der Nachreaktor wird über den vorlaufenden Wärmetauscher mit einer etwas höheren Temperatur derart beschickt, dass die gewünschte restliche Reaktion und Isomerenumformung zum dem finalen, gewünschten trans/trans-Verhältnis von bspw. 17 bis 23 Gew.% erfolgt. Der Nachreaktor mit der dortigen schwach exothermen, restlichen Reaktion wird hierbei weitgehend adiabat betrieben, was aber nicht im idealen Sinne zu verstehen ist.

Mit fortschreitender Zeit erfolgt ein Absinken der Katalysatoraktivität bis ein Austausch oder eine Regenerierung erforderlich ist. Hierzu wird parallel durch Regelung des Kühlkreislaufs die Betriebstemperatur angehoben, d.h. insb. eine geringere Kühlung über den im Kühlkreislauf eingebundenen Wärmetauscher vorgenommen, um Umsatz und Selektivität insgesamt auf einem weitgehend gleichbleibenden Niveau zu halten. Dies hat zur Folge, dass das Isomerenverhältnis verschoben wird, hinzu höheren trans/trans-Anteilen im Produkt. Hierbei hat es sich als sehr vorteilhaft erwiesen, dass der Stoffstrom im Feed des Nachreaktors über den vorlaufenden Wärmetauscher gegenläufig und eigenständig geregelt werden kann, insb. gleichläufig. So wird mit steigender Betriebstemperatur des Hauptreaktors über die Nutzungszeit des Katalysators die Feedtemperatur im Stoffstrom des Nachreaktors abgesenkt.

Vom Zeitpunkt t₀, dem Verfahrensstart nach Erneuerung oder Regeneration des Katalysators bis zum Zeitpunkt t₄, dem Ende des Verfahrens, bestimmt durch die Erneuerung oder Regeneration des Katalysators, kann die Betriebstemperatur des Hauptreaktors erhöht und Temperatur im Stoffstrom im Einlass (Feed) des Nachreaktors gleichgehalten oder gesenkt wird. Vorteilhafterweise ist die Anlage so ausgebildet, dass die Temperaturerhöhung oder -Absenkung kontinuierlich und/oder schrittweise erfolgen kann.

Der Hauptreaktor wird hierbei isotherm oder weitgehend isotherm und der Nachreaktor adiabat oder weitgehend adiabat betrieben. Zum Zeitpunkt t₀ (Verfahrensstart) kann neben dem Hauptreaktor auch der Katalysator im Nachreaktor erneuert oder regeneriert worden sein. Vorteilhafterweise wird der Zyklus zur Regeneration des Nachreaktors eigenständig und vom Hauptreaktor unabhängig bestimmt, insb. wenn o.g. Zusammenwirken der beiden Reaktoren nicht mehr die Produktion des gewünschten niedrigen trans/trans-Anteils im PACM ermöglicht.

Vorteilhafterweise ist die Mischereinheit zweiteilig ausgebildet und umfasst bspw. einen Mischapparat und einen Gas-Sättiger. Der Mischapparat kann insb. ein dynamischer oder statischer Mischer sein, der zur innigen Verbindung von MDA (Edukt1) mit dem Lösungsmittel geeignet ist. Der Gas-Sättiger kann insb. eine kleine Kolonne oder ein Kessel sein, sind dem geeignete Einbauten vorhanden sind, um das unter einem hohen Druck zugeführte H2-Gas, intensiv im MDA-Lösungsmittel-Stoffstrom zu lösen und/oder homogen vor dem Eintritt in den Reaktor zu verteilen. Der H2-Gasdruck beträgt vorteilhafterweise 70 bar bis 100 bar.

Mit dem Begriff "immobile Katalysatorpackung" oder "immobiler Katalysator" ist jede Form eines lokalen, nicht mit dem Stoffstrom strömenden oder fließenden Katalysator gemeint, wie insb. Katalysatorschüttungen (Pellets oder beschichtete Trägerkörper) oder festen Einbauten, die mit Katalysatormaterial beschichtet sind. Vorteilhafte Einbauteile, die eine Katalysatorbeschichtung aufweisen, können bspw. Gitter, Platten oder sonstigen Köper sein, die im Hauptreaktor angeordnet sind.

Im vorliegenden Zusammenhang meint eine "XY-einheit" und/oder "XY-stufe" immer auch mind. einen entsprechenden Apparat, Vorrichtung oder dergleichen, der/die von der jeweiligen Einheit oder Stufe umfasst ist. So meint bspw. eine Mischeinheit/-stufe, dass diese mind. einen Mischer/- vorrichtung umfasst.

Im vorliegenden Zusammenhang meint "Wärmetausch" oder "Wärmetauscher" immer einen indirekten Wärmetausch und entsprechende Bauformen mit geschlossenen Stoff- und Medienführungen, es sei denn, es ist ausdrücklich etwas hiervon Abweichendes beschrieben.

Vorliegend werden im Wesentlichen folgende Energiekopplungen (EK) betrachtet, wie eine integrierte Energiekopplung oder eine direkte Energiekopplung, wobei die integrierte EK eine integrierte Energiekopplung meint, unterteilt in
a. eine integrierte stoffbasierte Energiekopplung (isEK) von mind. zwei Stoffströmen in einem Wärmetauscher im indirekten Wärmeaustausch meint, d.h. eine bauliche Integration in einem einzigen Wärmetauscher (Apparat), d.h. wo vormals zwei Wärmetauscher vorgesehen waren, sind beide Wärmetransportaufgaben einer baulichen Einheit (Wärmetauscher) integriert,
b. eine integrierte medienbasierte Energiekopplung (integrierte medienbasierte EK) von mind. zwei Wärmetauschmedien in einem Wärmetauscher im indirekten Wärmeaustausch meint, d.h. eine bauliche Integration in einem einzigen Wärmetauscher (Apparat);

Die vorgenannten EK können als direkte Energiekopplung (direkte EK) ausgestaltet sein, indem eine serielle EK oder serielle Verschaltung von mind. zwei Wärmetauschern vorgesehen ist.

Mit der "Kondensationseinheit" der ersten Trennstufe ist ein einzelner Wärmetauscher oder eine Gruppe von Wärmetauschern bezeichnet, die zur mind. teilweisen Kondensation und/oder Abkühlung der über die Kopfleitung/en abgeleiteten Anteile an Leichtsiedern dienen. Eine derart bezeichnete "Kondensationseinheit" muss keine (geschlossene) Baueinheit darstellen. Somit wird zum Teil der Begriff "Kondensationseinheit" und einen einzelner "Wärmetauscher" auch synonym verwendet.

Die als "erste Trennstufe" bezeichnete Stufe ist insb. dadurch bestimmt und weist entsprechende Apparate und Leitungen auf, dass das Lösungsmittel (gezielt) vom produktreichen Stoffstrom abgetrennt wird und vorteilhafterweise auch zur Verwendung in die Reaktoreinheit und/oder die Konditionierungseinheit zurückgeleitet wird. In analoger Weise meint die als "zweite Trennstufe" bezeichnete Stufe der Trenneinheit, dass diese dadurch bestimmt ist und entsprechende Apparate und Leitungsführungen aufweist, um das Produkt von Nebenprodukten und Edukten, insb. MDA zu trennen und das Produkt aufzureinigen. Hierbei sind die erste und zweite Stufe naturgemäß nicht ganz streng getrennt und es kann ein Überlappungsbereich oder ein Apparat im Übergangsbereich vorhanden sein, in dem sowohl Lösungsmittel als auch mind. ein Nebenprodukt oder mind. ein Edukt vom Produkt getrennt wird. In dem vorliegenden Zusammenhang meint die "Abtrennung von Lösungsmittel" in der ersten Trennstufe und "Abtrennung von mind. einem Edukt und/oder mind. einem Nebenprodukt vom Produkt", wobei das Produkt inb. PACM sein kann, dass die Abtrennung keine absolute Abgrenzung der Trennstufen meint, sondern jeweils "im Wesentlichen" nur den/die genannten Stoff/e betrifft.

Der von einem Druckentspannungstrennkessel, nachstehend Trennkessel genannt (auch teilweise Flashbehälter genannt), kommende flüssige Stoffstrom wird über eine Leitung zur ersten Trennkolonne innerhalb der ersten Trennstufe der Trenneinheit geleitet. Der Trennkessel zeichnet sich dadurch aus, dass durch Entspannung (Druckabsenkung) der zufließende Stoffstrom in eine Dampfphase (Lösungsmittel, lösungsmittelreich) und eine Flüssigphase (verarmt an Lösungsmittel) aufgetrennt wird und im Regelbetrieb im Trennkessel beide Phasen vorliegen. Der Trennkessel kann zusätzlich einen Sumpfumlauf mit integriertem Wärmetauscher aufweisen oder hieran angeschlossen sein, um durch Erwärmung der Flüssigphase den abtrennbaren Dampfanteil über den druckbedingten Anteil hinaus zu erhöhen. Weiterhin kann ein Trennkessel Einbauten oder Füllkörper umfassen, insb. um das Mitreißen von nicht oder nur unvollständig an Lösungsmittel verarmten Tröpfchen zu verhindern. Vorteilhafterweise kann bei einer Ausführungsform der Anlage vorgesehen sein, dass in der Leitung vom Trennkessel zur ersten Kolonne eine Druckregeleinheit vorgesehen ist. Hierdurch kann die Reaktoreinheit auf einem ersten, hohen Druckniveau betrieben werden und die ersten Trennstufe der Trenneinheit auf einem zweiten, tieferen Druckniveau. Im vorliegenden Zusammenhang meint also Trennkessel (Flashbehälter) einen Apparat, indem im Wesentlichen durch Druckentspannung eine Phasentrennung veranlasst wird. Trennkolonne meint vorliegend hingegen einen Apparat, in dem im Wesentlichen durch Energiezufuhr einen Trennung in eine Dampfphase und Flüssigphase erfolgt, insb. unter Einbindung eines Kopfumlaufs, indem im Kopf mind. ein Teil der auskondensierten Flüssigkeit zurück in die Kolonne geleitet wird.

Der Begriff "Eduktmischung" meint in dem vorliegenden Zusammenhang die Mischung aus Stoffen, die beim Eintritt in den (ersten) Hauptreaktor vorliegt, also die Mischung aus allen Edukten, Lösungsmitteln, Hilfsstoffen etc. in und nach dem mind. einen Reaktor wird die strömende Mischung aus Stoffen in jedem Grad der Reaktion oder nachfolgenden Aufreinigung als "Stoffstrom" oder "Stoffmischung" (auch "Stoffgemisch") bezeichnet, wobei zum Teil adjektivische Beschreibungen wie "produktreich" oder "lösungsmittelreich" angefügt sind. Die stoffliche Zusammensetzung des Stoffstroms an jedem Ort der Anlage ergibt sich für den Fachmann allerdings auch in naheliegender Weise durch ebendiesen Anlagenort und stromaufwärts befindliche Anlagenkomponenten und insb. die verfahrenstechnischen Apparate der Anlagen. Die Reinstoffe, wie bspw. das Produkt, insb. das Produkt PACM, sowie die Nebenprodukte LB und HB, sind gesondert benannt und ausgewiesen. Hierbei steht "LB" für "Low Boiler", einem werthaltigen Stoffgemisch, das gesondert aus dem Stoffstrom und vom Produkt abgetrennt wird und einen niedrigen Siedepunkt von ca. 240 °C bis 290 °C aufweist. In analoger Weise steht "HB" für "High Boiler", einem werthaltigen Stoffgemisch, das gesondert aus dem Stoffstrom und vom Produkt abgetrennt wird und einen hohen Siedepunkt von > 350 °C aufweist.

Vorliegend wird insb. eine Anlage und ein Verfahren zur Produktion von Methylenbis(cyclohexylamin) als Produkt, insb. zur Produktion von 4,4'-Diaminodicyclohexylmethan (PACM), durch katalytische Hydrierung von Methylendianilin beansprucht. Hierbei ist das primäre Produkt PACM, das aus 4,4`-Diaminodiphenylmethan (4,4`-MDA; primärer Anteil des Edukts1) hydriert wird, insb. mit dem genannten niedrigen trans/trans-Isomerenverhältnis, so dass die Anlage und das Verfahren insb. zur Produktion von 4,4`-Diaminodicyclohexylmethan (PACM) durch kontinuierliche, katalytische Hydrierung von 4,4'-Diaminodiphenylmethan (4,4'-MDA) dient und geeignet ist. Die weiterhin vorliegenden Anteile 2,4'-MDA und 2,2'-MDA des MDA werden mind. zu geringen Anteilen parallel zu Reaktionsprodukten gewandelt, wobei diese in der Regel in der Produktmischung verbleiben. Weiterhin können vorteilhafterweise ebenfalls durch dieses Verfahren bzw. diese Anlage in der zweite Trennstufe der Trenneinheit sonstige Nebenprodukte aufgereinigt und abgetrennt werden, insb. in mind. einer Trennkolonne. Diese stellen regelmäßig sekundäre (werthaltige) Produkte dar und sind hierin im Wesentlichen als High Boiler (HB) und Low Boiler (LB) beschrieben und gemeint.

Vorteilhafterweise ist das Lösungsmittel aus der nachfolgenden Gruppe von Stoffen: Cyclohexan, Dioxan, Tetrahydrofuran (THF), Cyclohexylamin, Dicyclohexylamin, Methanol, Ethanol, Isopropanol, n-Butanol, 2-Butanol, 2-Methoxy-2-methylpropan (MTBE) oder Methylcyclohexan oder ein Gemisch hieraus. Vorteilhafterweise wird das Lösungsmittel, insb. THF im Überschuss zum MDA zugeführt, so dass vorteilhafterweise das Verhältnis der Massenströme von Lösungsmittel, insb. THF, zu MDA am Einlass des Hauptreaktors im Bereich von 1,0 bis 8,0 liegt, insbesondere im Bereich von 2 bis 7,5.

Bei einer weiteren vorteilhaften Ausführungsform der Anlage kann vorgesehen sein, dass in dem mind. einen hinführenden Leitungsast des Verteilerkreislaufs mind. ein Verdichter eingebunden ist. Hierbei besteht eine Verbesserung darin, dass eine mehrstufige Verdichtung vorgesehen wird, also eine Gruppe aus mindestens zwei Verdichtern in dem hinführenden Leitungsast vorgesehen werden. Die Verdichtung wirkt auf den vom Sammelkessel kommenden Dampf ein.

Auch wenn die Verdichtung von einem Anfangsdruck bei einer Anfangstemperatur, wie sie im Verteilerkessel vorliegen, auf einen gewünschten Enddruck bzw. Endtemperatur grundsätzlich auch einstufig erfolgen kann, besteht der Vorteil einer mehrstufigen Verdichtung darin, dass Zwischeneinspeisungen, Abzweigungen bei Zwischendrücken und Zwischentemperaturen möglich sind. Weiterhin sinkt der Verbrauch an elektrischer Leistung, wenn nur noch auf einen bereits vorverdichteten Volumenstrom durch einen nachfolgenden Verdichter eingewirkt werden muss und die Verdichter können baulich kleiner ausgebildet sein, als ein einstufiger (End-)Verdichter.

Bei einer weiteren vorteilhaften Ausführungsform der Anlage kann vorgesehen sein, dass der Verteilerkreislauf mind. zwei (Teil-)Kreisläufe umfasst,
- mind. einen Hochdruck-Verteilerkreislauf (HD-Verteilerkreislauf), der an einen Kopf- oder Dampfauslass des Verteilerkessels angeschlossen ist und in den mind. ein Verdichter eingebunden ist und
- mind. einen Niederdruck-Verteilerkreislauf (ND-Verteilerkreislauf), der an einen Kopf-/Dampfauslass des Verteilerkessels angeschlossen ist und in den kein Verdichter, weniger Verdichter und/oder weniger Verdichterleistung als in den HD-Verteilerkreislauf eingebunden sind, und wobei
in den HD-Verteilerkreislauf und den ND-Verteilerkreislauf als Wärmesenke mind. ein Wärmetauscher der Konditionierungseinheit, Trenneinheit und/oder der Reaktionseinheit wärmetauschend eingebunden sind, und wobei der Zwischenkreislauf und der Verteilerkreislauf über den Verteilerkessel medienleitend miteinander verbunden sind.

Hierbei meint, dass in den ND-Verteilerkreislauf in einer Ausführungsform "weniger Verdichter und/oder weniger Verdichterleistung" eingebunden sind, als in den HD-Verteilerkreislauf, dass der mögliche Enddruck und damit auch die Endtemperatur (ohne weiteren Wärmetausch) aufgrund der Anzahl und/oder der Bauart der Verdichter im ND-Verteilerkreislauf niedriger ist als in dem HD-Verteilerkreislauf. Anders ausgedrückt, ein niedrigerer Druck des erzeugten Dampfes als im HD-Kreislauf ist hinreichend, um die Heizaufgaben im ND-Verteilerkreislauf auf niedrigerem Temperaturniveau zu erfüllen.

Unter dem HD-Verteilerkreislauf ist vorliegend der (Teil-)Kreislauf des Verteilerkreislaufes zu verstehen, in dem (innerhalb der Anlage) der maximal mögliche (End-)Druck und damit in der Regel auch höchste Endtemperatur des Verteilerkreislaufs im dampfförmigen Medium erzeugbar ist, was aufgrund der Leistung und/oder Anzahl an (insb. seriell zueinander geschalteten) Verdichtern und/oder der hiermit möglichen Verdichterleistung erfolgen kann.

Bei dieser Ausführungsform umfassen der HD-Verteilerkreislauf und der ND-Verteilerkreislauf des Verteilerkreislaufs jeweils mind. einen Leitungsast oder -abschnitt zur Verteilung- oder Abgabe von Energie, wobei
- in den ND-Verteilerkreislauf ein erster Wärmetauscher oder eine erste Gruppe aus zwei oder mehr Wärmetauschern eingebunden ist, und wobei
- in den HD-Verteilerkreislauf ein weiterer Wärmetauscher oder eine weitere Gruppe aus zwei oder mehr Wärmetauschern eingebunden ist. Auf diese Weise kann der erste Wärmetauscher oder die erste Gruppe aus zwei oder mehr Wärmetauschern des ND-Verteilerkreislaufs auf einem ersten Temperatur- und Druckniveau betrieben werden und der weitere Wärmetauscher oder die weitere Gruppe aus zwei oder mehr Wärmetauschern des HD-Verteilerkreislaufs auf einem zweiten, höheren Temperatur- und Druckniveau.

Hierbei ist weiterhin in einer rückführenden (Sammel-)Leitung mind. eine Druckregeleinheit vorgesehen, insb. für jedes Druckniveau eine Druckregeleinheit (Entspannungseinheit) vorgesehen, bis zur Einleitung in den Sammelkessel oder eine zentrale, rückführende Leitung, die in den Sammelkessel einleitet.

Weiterhin ist mit dem Anschluss "an einen Kopf- und/oder Dampfauslass des Verteilerkessels" gemeint, dass dieser Anschluss in einer gemeinsamen (zentralen) Leitung bestehen kann, von der Abzweigungen in den HD- oder ND-Niederdruckkreislauf bestehen, oder beide Kreisläufe jeweils einzeln separat Anschlüsse an dem Verteilerkessel aufweisen.

Bei einer weiteren vorteilhaften Ausführungsform der Anlage kann vorgesehen sein, dass im Zwischenkreislauf im hinführenden Leitungsast eine Gebläse oder eine Gruppe aus mind. zwei Gebläsen eingebunden ist. Es hat sich als vorteilhaft erwiesen, den gewünschten Druckanstieg mehrstufig auszuführen, weil hierdurch insgesamt der Stromverbrauch sinkt, da die nachfolgenden Verdichter auf einen immer geringeren Dampfvolumenstrom einwirken müssen, wodurch sie baulich kleiner ausgeführt werden können. Hierbei kann im Zwischenkreislauf eine Gruppe von x Gebläsen eingebunden sein, wobei x eine ganzzahlige Zahl von größer oder gleich 2 ist, insb. größer oder gleich 3. Die Gebläse sind zueinander in Serie geschaltet, so dass eine mind. zweistufige Dampfverdichtung im hinführenden Leitungsast erfolgt. In der Regel ist es nicht vorteilhaft, mehr als 5 oder 6 Gebläse in Reihe vorzusehen. Unter einem "Gebläse" ist hierbei eine Strömungsmaschine zur Gas- oder Dampfverdichtung zu verstehen, die ein Verhältnis TT von Enddruck (Druckseite, Ausgang) zum Eingangsdruck (Saugseite, Eingang) von 1,3 bis 2,5 hat. Strömungsmaschinen mit TT » 2,5 sind vorliegend mit "Verdichter" gemeint.

Bei einer weiteren vorteilhaften Ausführungsform der Anlage kann vorgesehen sein, dass in den Medienumlauf des Sammelkreislaufs ein Sammeltank und eine Pumpe eingebunden sind, wobei ein Wärmetauscher stromaufwärts zum Sammeltank und/oder auf der Saugseite der Pumpe angeordnet ist. Bei einer Ausführungsform ist der Wärmetauscher in den Sammeltank integriert und/oder der Sammeltank weist eine integrierte Temperiereinheit auf. Der Vorteil hierbei ist, dass unabhängig vom aktuellen Energieeintrag in den Sammelkreislauf und/oder der aktuellen Energieentnahme am eingebundenen ersten Verdampfer, im hinführenden Leitungsast insgesamt das erforderliche Temperaturniveau und der erforderliche Volumenstrom an Medium bereitgestellt werden kann. Das zirkulierte Wärmetauschmedium des Sammelkreislaufs ist vorteilhafterweise Wasser oder eine im Wesentlichen wässrige Lösung.

Bei einer weiteren vorteilhaften Ausführungsform kann vorgesehen sein, dass der Verteilerkreislauf einen ersten Leitungsast als dampfführende Zuleitung umfasst, wobei in diesen Leitungsast mind. ein Verdichter eingebunden ist, idealerweise eine Gruppe von Verdichtern vorgesehen ist, die zwei bis fünf Verdichter umfasst, insb. zwei oder drei Verdichter umfasst. Die zwei oder mehr Verdichter sind vorteilhafterweise zueinander in Serie geschaltet.

Auf diese Weise kann mit hohem Wirkungsgrad die Temperatur des vom Verteilerkessel kommenden Dampfes in der hinführenden Leitung um 30 bis 150 °C angehoben werden, insb. um 70 bis 120 °C angehoben werden. Insgesamt ist der Verteilerkreislauf ein Wasserkreislauf, bzw. ein Kreislauf, dessen Wärmetauschmedium Wasser oder eine wässrige Lösung ist.

Bei einer weiteren vorteilhaften Ausführungsform der Anlage kann vorgesehen sein, dass bei zwei oder mehr Verdampfern in dem hinführenden Leitungsast, vom Verteilerkessel mind. eine (SumpfLeitung und/oder mind. einen Abzweigung einer (Sumpf-)Leitung auf die Druckseite mind. eines Verdichters führt, wobei in die (Sumpf-)Leitung eine Pumpe eingebunden ist.

Vorteilhafterweise ist bei einer Ausführungsform zwischen ein Paar aus zwei Verdichtern, insb. jeweils zwischen jedes Paar aus zwei seriellen Verdichtern jeweils eine (Sumpf-)Leitung und/oder mind. eine Abzweigung einer (Sumpf-)Leitung geführt. Es hat sich überraschenderweise als insgesamt energetisch vorteilhaft herausgestellt, dass das auf einem etwas niedrigeren Temperaturniveau befindliche flüssige Medium der (Sumpf-)Leitung in den hinführenden (Dampf-)Leitungsast einzuspeisen. Die Druckerhöhung mittels Pumpe in dem flüssigen Medium der (Sumpf-)Leitung und Entspannung über eine Druckregeleinheit ist energetisch vorteilhaft gegenüber der direkten Dampfverdichtung, so dass der Energiebedarf bzw. die Leitungsaufnahme des stromaufwärts zur Einspeisung angeordneten Verdichters verringert wird, weiterhin wird auch der Energiebedarf des jeweils stromabwärts befindlichen Verdichters verringert, weil durch die mit der Zwischeneinspeisung verbundene Dampfabkühlung eine Volumenreduktion des verdichteten Dampfes (vom vorlaufenden Verdichter) bei gleichzeitiger Erhöhung des Massestromes durch den entspannten, verdampften Medienzustrom (aus der Zwischeneinspeisung) erfolgt.

Vorteilhafterweise ist hierzu in der (Sumpf-)Leitung bzw. in den jeweiligen Abzweigungen von der (Sumpf-)Leitung ein regelbares Ventil bzw. eine Druckregeleinheit vorgesehen, um die Volumenströme zu regeln bzw. vollständig abzustellen. Insb. wird über das regelbare Ventil bzw. die Druckregeleinheit sichergestellt, dass das flüssige Medium aus dem Verteilerkessel und der Sumpfleitung, dessen Druck über eine Pumpe einstellbar ist, verdampft und dampfförmig in den jeweiligen Abschnitt des hinführenden Leitungsastes eingeleitet wird.

Bei einer weiteren vorteilhaften Ausführungsform der Anlage kann vorgesehen sein, dass die (Sumpf-)Leitung mind. zwei Äste aufweist, wobei jeder Ast der (Sumpf-)Leitung zwischen zwei der Verdichter des hinführenden Leitungsastes des HD-Verteilerkreislaufs geführt ist. Die in der (Sumpf-)Leitung arbeitende Pumpe ist vorteilhafterweise stromabwärts zum Verteilerkessel und stromaufwärts zur ersten Abzweigung oder Leitungsast Ästen angeordnet.

Vorteilhafterweise sind in mind. einem Ast der (Sumpf-)Leitung Druck- und/oder Temperatursensoren vorgesehen.

Bei einer weiteren vorteilhaften Ausführungsform der Anlage kann vorgesehen sein, dass in mind. einem rückführenden Leitungsast des Verteilerkreislaufs ein Wärmetauscher wärmetauschend eingebunden ist. Dieser Wärmetauscher ist stromabwärts zum Leitungsast zur Verteilung in den rückführenden Leitungsast eingebunden. Dieser Wärmetauscher ist ein Bedarf- oder Regelwärmetauscher, über den sichergestellt werden kann, dass unabhängig vom Energieverbrauch bzw. der Energieabgabe im Leitungsast zur Verteilung, dem Verteilerkessel Medium auf dem erforderlichen Temperaturniveau zur Verfügung gestellt wird. Dieser Bedarfs- oder Regelwärmetauscher ist vorteilhafterweise keinem sonstigen Apparat der Anlage zugeordnet, d.h. ist nicht vorgesehen, eine sonstige Wärmetauschaufgabe zu erfüllen, als den Vorlauf zum Verteilerkessel zu regeln.

Bei einer weiteren vorteilhaften Ausführungsform der Anlage kann vorgesehen sein, dass der ND-Verteilerkreislauf des Verteilerkreislaufs mind. zwei Teilkreisläufe zur Verteilung- oder Abgabe von Energie umfasst, wobei
- der erste Teilkreislauf ein Niederdruckkreislauf (ND-Teilkreislauf) ist, wobei in den ersten ND-Teilkreislauf mind. ein Wärmetauscher oder eine erste Gruppe aus zwei oder mehr Wärmetauschern eingebunden ist, und wobei
- in den weiteren Teilkreislauf mind. ein Wärmetauscher oder eine weitere Gruppe aus zwei oder mehr Wärmetauschern eingebunden ist, wobei in den ND-Teilkreislauf kein Verdichter, weniger Verdichter und/oder eine geringere Verdichterleistung eingebunden ist, als in jeden weiteren Teilkreislauf des ND-Verteilerkreislaufs.

Jeder Teilkreislauf weist hierbei eine zuführende Leitung auf. Die jeweilige zuführende Leitung kann jeweils eigenständige Verdichter oder Verdichterstufen aufweisen. Vorteilhafterweise zweigt die jeweilige zuführende Leitung für einen Teilkreislauf des ND-Verteilerkreislaufs bei einer unterschiedlichen Verdichterstufe von der zuführenden Leitung des HD-Verteilerkreislaufs ab. So kann beispielsweise zur zuführenden Leitung des ND-Teilkreislaufes auf der niedrigsten, ersten Verdichterstufe nach dem ersten Verdichter abzweigen, die zum zweiten Teilkreislauf des ND-Verteilerkreislaufs abzweigende, zuführende Leitung, zweigt nach dem zweiten Verdichter der zuführenden Leitung des HD-Verteilerkreislaufs ab. Somit haben die Teilkreisläufe unterschiedliche, ansteigende Druck- und Temperaturniveaus. Dies ermöglicht, dass genau der Wärmetauscher bzw. die Gruppe von Wärmetauschern als Wärmesenke eingebunden werden können, für die das jeweilige Temperaturniveau hinreichend ist, so dass Energieverbrauch für weitergehende Verdichtung nur gezielt für die Wärmetauscher erfolgen braucht, die ein erhöhtes Temperaturniveau benötigen.

Analog zu der Definition des HD-Verteilerkreislaufs meint vorliegend "ND-Teilkreislauf" denjenigen Teilkreislauf, der auf dem geringsten Druckniveau aller Teilkreisläufe des ND-Verteilerkreislaufes betreibbar ist, weil ausgehend vom Verteilerkessel in der hinführenden Leitung kein Verdichter, weniger Verdichter und/oder eine geringere Verdichterleistung eingebunden ist, als in jeden weiteren Teilkreislauf, wobei dies auf den Regelbetrieb der Anlage bezogen ist. Ausfall- oder Schadenssituationen sollen hierbei nicht gemeint sein.

Die Teilkreisläufe umfassen weiterhin jeweils einen weiteren Leitungsast zur Rückführung des Mediums zum Verteilerkessel oder sind mit einer zentralen, gemeinsamen rückführenden Leitung verbunden, die am Verteilerkessel angeschlossen ist und so das an Energie verarmte Medium in den Verteilerkessel leitet.

Bei einer vorteilhaften Ausführungsform sind
- in den HD-Verteilerkreislauf mind. zwei Wärmetauscher aus Sumpfumläufen von zwei Trennkolonnen der Trenneinheit eingebunden, wobei eine dreistufige Verdichtung in der hinführenden Leitung vorgesehen ist,
- in den ersten Teilkreislauf des ND-Verteilerkreislaufs der (Sumpf-)Wärmetauscher eines Trennkessels der Trenneinheit eingebunden, wobei eine einstufige Verdichtung bis zum Abzweig der hinführenden Leitung vorgesehen ist, und wobei
- in dem zweiten Teilkreislauf des ND-Verteilerkreislaufs zwei Wärmetauscher aus Sumpfumläufen von zwei Trennkolonnen der Trenneinheit eingebunden, wobei eine zweistufige Verdichtung bis zum Abzweig in der hinführenden Leitung vorgesehen ist.

Vorteilhafterweise erfolgt stromabwärts zu jedem Abzweig einer hinführenden Leitung zu einem der beiden Teilkreisläufe des ND-Verteilerkreislaufes eine Entspannung und Zwischeneinspeisung von Medium aus einer (Sumpf-)Leitung, die mit dem Sammelkessel verbunden ist.

Die (Teil-)Gruppen von zwei oder mehr Wärmetauschern, die als Wärmesenken in einen der Verteilerkreisläufe, auch Teilkreisläufen, bei einem jeweiligen Druckniveau eingebunden sind, sind parallel zueinander geschaltet. Die Zuleitungen zu jedem der Wärmetauscher einer Gruppe zweigen vorteilhafterweise von einer gemeinsamen Verteilerleitung ab und die Ableitungen von jedem Wärmetauscher einer Gruppe mündet vorteilhafterweise in eine gemeinsame Sammelleitung. Mehrere Gruppen von Wärmetauschern können über eine gemeinsame Sammelleitung abgeleitet werden, wobei zur Abgrenzung der Druckniveaus mind. eine Druckregeleinheit zur Druckabsenkung stromabwärts zu jeder Gruppe von Wärmetauschern und/oder jedem Teilkreislauf in der jeweiligen Sammelleitung und/oder der jeweiligen rückführenden Leitung vorgesehen ist.

Mehrere Sammelleitungen können in einen gemeinsame, rückführende Leitung münden, die an den Verteilerkessel angeschlossen ist.

Bei einer weiteren vorteilhaften Ausführungsform der Anlage kann vorgesehen sein, dass der Verteilerkreislauf einen weiteren Leitungsast zur Verteilung- und/oder Abgabe von Energie und einen weiteren Leitungsast zur Medienrückführung umfasst, wobei in den Leitungsast zur Verteilung mind. ein Wärmetauscher der Reaktoreinheit, mind. ein Wärmetauscher der Konditionierungseinheit und/oder mind. ein Wärmetauscher der Trenneinheit als Wärmesenke eingebunden ist, insb. eine Mehrzahl von den jeweiligen Wärmetauschern eingebunden sind. Bei einer besonders bevorzugten Variante sind alle weiteren Leitungsäste zur Verteilung- und/oder Abgabe von Energie mit jeweils einem Abzweig von dem hinführenden Leitungsast verbunden, die mit unterschiedlichen Druckniveaus und Temperaturniveaus korrelieren. So kann ein erster Abzweig stromabwärts zum ersten Verdichter, ein zweiter Abzweig stromabwärts zum zweiten Verdichter etc. angeordnet und/oder ein erster Abzweig stromabwärts zu einer ersten Druckregeleinheit, ein zweiter Abzweig stromabwärts zur einer zweiten Druckregeleinheit etc.

Durch diese Gruppierung der Wärmetauscher durch weitere Leitungsäste können einzelne Wärmetauscher oder Gruppen aus zwei oder mehr Wärmetauschern gezielt nach Energiebedarf und nach Bedarf des Temperaturniveaus beschickt werden. So kann vorteilhafterweise ein Wärmetauscher oder eine Gruppe aus Wärmetauschern mit einem niedrigen Temperaturniveau und/oder einer hohen Austauschleistung in Strömungsrichtung zuerst ausgeleitet werden, so dass der Stromverbrauch der nachfolgenden Verdichter entsprechend gesenkt wird. Bedarfsweise kann Medium aus der (Sumpf-)Leitung in dem hinführenden Leitungsast zugeführt werden, insb. in dampfförmiger Phase zugeführt werden, wie vorstehend beschrieben.

Bei einer weiteren vorteilhaften Ausführungsform der Anlage kann vorgesehen sein, dass bei mehr als einem rückführenden Leitungsast oder mind. einer Sammelleitung des Verteilerkreislaufs und/oder eines HD-, ND- oder Teilkreislaufs, ein Bedarf- und/oder Regelwärmetauscher in mind. einem weiteren rückführenden Leitungsast wärmetauschend eingebunden ist, idealerweise in allen rückführenden Leitungsästen ein solcher Bedarf- und/oder Regelwärmetauscher eingebunden ist. Diese sind vorteilhafterweise an einem unabhängigen Wärme- und/oder Kühlkreislauf und/oder eine unabhängige Wärme- und/oder Kühlquelle angeschlossen, so dass hierüber bedarfsweise bei Anfahr-, Wartungs- und/oder Unterbrechungssituationen die erforderliche Betriebstemperatur in dem Zwischen- und/oder Verteilerkreislauf hergestellt werden kann.

Bei einer weiteren vorteilhaften Ausführungsform der Anlage kann vorgesehen sein, dass mind. eine Teilzahl der als Wärmesenke eingebundenen Wärmetauscher des Verteilerkreislaufs parallelgeschaltet sind. Vorteilhafterweise sind die einzelnen Wärmetauscher oder die Gruppen aus parallelgeschalteten Wärmetauschern jeweils im Zu- oder Ablauf steuer- und/oder regelbar. Hierbei betrifft die Regelbarkeit insb. die jeweiligen Durchflussmengen an Medium, wobei diese Regelung vorteilhafterweise in Abhängigkeit von dem erforderlichen Temperaturgradienten im jeweiligen Wärmetauscher bzw. der Gruppe von Wärmetauschern und/oder dem erforderlichen Energieübertrag erfolgt. Bei einer weiteren vorteilhaften Ausführungsform ist ein einzelner als Wärmesenke dienender Wärmetauscher bzw. eine Gruppe aus zwei oder mehr Wärmetauschern nicht in den Verteilerkreislauf eingebunden. Die Einbindung dieser Wärmetauscher, insb. (Sumpf-)Wärmetauscher der Trennkolonnen aus der Trenneinheit, hätten bei der Einbindung in den Verteilerkreislauf einen elektrischen Strombedarf für die Verdichter und/oder einen weiteren Verdichter, der vergleichbar einer direkten, elektrischen Beheizung dieser Wärmetauscher ist. Es hat sich als insgesamt als vorteilhaft erwiesen, wenn bereits bei einem erhöhten Energiebedarf für die direkte, elektrische Beheizung von Wärmetauschern von Faktor 1,2 bis 1,3 gegenüber der Einbindung in den Verteilerkreislauf, aufgrund geringerer sonstiger Aufwände, wie eines geringeren Verschleißes, geringerer Stillstandzeiten, etc. eine elektrische Direktbeheizung vorteilhaft ist.

Bei einer weiteren vorteilhaften Ausführungsform der Anlage kann vorgesehen sein, dass die als Wärmequelle wirkenden Wärmetauscher des Sammelkreislaufs in Reihe geschaltet sind. Hierzu sind die eingebundenen Wärmetauscher in Strömungsrichtung des hinleitenden Leitungsastes vorteilhafterweise mit aufsteigendem Temperaturniveau in diesen Leitungsast eingebunden. Als die zentrale Wärmequelle für den Sammelkreislauf dient der im Kühlkreislauf des Hauptreaktors eingebundene Wärmetauscher, der zur Ableitung der exothermen Energie aus der Reaktion dient. Als weitere Wärmequellen dienen insb. die (Kopf-)Wärmetauscher (Kondensatoren) der Trennkolonnen, abhängig von dem dortigen Temperaturniveau im Medium des jeweiligen Wärmetauschers.

Der von dem Trennkessel kommende flüssige Stoffstrom wird über eine (Feed-)Leitung zur ersten Trennkolonne der ersten Trennstufe der Trenneinheit geleitet. Vorteilhafterweise kann bei einer Ausführungsform der Anlage vorgesehen sein, dass in der (Feed-)Leitung vom Trennkessel zur ersten Kolonne eine Druckregeleinheit vorgesehen ist. Hierdurch kann die Reaktoreinheit auf einem ersten, hohen Druckniveau betrieben werden und die erste Trennkolonne der ersten Trennstufe auf einem zweiten, tieferen Druckniveau, wobei der Trennkessel auf einem Zwischenniveau betrieben werden kann.

Weiterhin kann es vorteilhaft sein, wenn stromaufwärts zur ersten Trennkolonne ein Wärmetauscher vorgesehen ist, insb. auch stromabwärts der Druckregeleinheit bzw. zwischen der Druckregeleinheit und der ersten Trennkolonne.

Bei einer besonders vorteilhaften Ausführungsform ist eine Energiekopplung vorgesehen, um den Kondensator stromabwärts zum Trennkessel, einen Kondensator einer Trennkolonne der zweiten Trennstufe oder den (Umlauf-)Wärmetauscher im Medienumlauf des mind. einen Hauptreaktors verschaltet im Wärmetausch mit dem (Vorlauf-)Wärmetauscher der ersten Trennkolonne zu betreiben. Die Energiekopplung kann durch Medienleitung und eine serielle Verschaltung der jeweiligen Wärmetauscher erfolgen oder durch eine integrierte Energiekopplung in einem (baulich) einzigen Wärmetauscher. Die integrierte Energiekopplung hat den Vorteil, sofern räumlich innerhalb der Anlage realisierbar, dass nur ein Temperaturgefälle für den Wärmetransport überwunden werden muss. Durch Energieübertrag kann so der (Feed-)Stoffstrom vor der ersten Trennkolonne der ersten Trennstufe, der nach der stromaufwärtsbefindlichen Druckregeleinheit in der (Feed-)Leitung ein Temperaturniveau von ca. 85 bis 95 °C aufweist, parallel angehoben werden. Da der Hauptreaktor im Laufe des Betriebes bei stetig steigender Temperatur betrieben wird, um die sinkende Katalysatoraktivität zu kompensieren, kann parallel eine stetig steigende Energiemenge an den (Feed-)Stoffstrom vorlaufend der ersten Trennkolonne abgegeben werden. Hierdurch sinkt ebenfalls stetig der Energiebedarf im Sumpfkreislauf, bzw. dem dortig eingebundenen Wärmetauscher der ersten Trennkolonne.

Bei einer Ausführungsform der Anlage, kann es vorteilhaft sein, dass die Reaktoreinheit einen ersten Hauptreaktor und mind. einen stromabwärts befindlichen permanenten, in Serie geschalteten Nachreaktor umfasst. Der besondere Vorteil des Nachreaktors und dessen eingangsseitige Temperierung des Stoffstroms besteht darin, dass auf diese Weise eine optimierte Steuerung der Selektivität der Anteile an Isomeren ermöglicht wird, aufgrund der vom ersten Reaktor abweichenden, bevorzugt höheren Eintrittstemperatur.

Vorteilhafterweise ist der Nachreaktor auch ein Festbettreaktor, bzw. Reaktor mit einem immobilen Katalysator, wie beispielsweise einer Katalysatorschüttung oder mit Katalysator beschichteten Einbauten.

Vorteilhafterweise umfasst der immobile Katalysator Ruthenium, ist mit Ruthenium dotiert oder ist hieraus gebildet. Bei einer vorteilhaften Verfahrensvariante, insb. zur Erreichung eines niedrigen Anteils an trans/trans-Isomeren im Isomerengemisch, wird der Hauptreaktor bei einer Temperatur von 90 bis 140 °C, idealerweise 95 bis 135 °C.

Es hat sich als besonders vorteilhaft herausgestellt, wenn das Verhältnis der Katalysatormassen vom Hauptreaktor zum Nachreaktor ist im Bereich von 1,2 bis 2 liegt, bevorzugt 1,3 zu 1,4 und idealerweise 1,35. Es hat sich überraschenderweise gezeigt, dass es hinreichend ist, die stark exotherme Reaktion beim Reaktionsstart im Hauptreaktor durch einen intensiven Kühlkreislauf zu regeln, und lediglich die Vorlauftemperatur im Zulauf zum Nachreaktor so einzustellen, dass der moderate Temperaturanstieg von 30 bis 35 °C im Nachreaktor vom Einlass zum Auslass nur noch einen limitierten und gut reproduzierbaren Einfluss auf den trans/trans-Isomerenanteil im Stoffstrom bzw. im Produkt hat, wie bereits ausgeführt.

Es kann insb. vorteilhaft sein, wenn der Hauptreaktor ein Festbettreaktor ist, der
- einen ersten Strömungsweg für das Edukt- bzw. Stoffgemisch und
- einen weiteren (geschlossenen) Strömungsweg, d.h. einem Medienumlauf für ein Wärmetauschmittel umfasst, wobei in den zweiten Strömungsweg zwei Wärmetauscher für einen indirekten Wärmeaustausch eingebunden sind:
   - einen als Kühler arbeitenden (Haupt-)Wärmetauscher und
   - einen als Heizer arbeitenden (Neben-)Wärmetauscher.

Es hat sich als sehr effektiv und vorteilhaft herausgestellt, denselben weiteren Strömungsweg, bzw. Medienumlauf zum Vorbereiten und Anfahren des Hauptreaktors mittels (Neben-)Wärmetauschers zu betreiben und die Kühlung der Hauptreaktion im produzierenden Betrieb des Hauptreaktors mittels des (Haupt-)Wärmetauschers zu betreiben.

Bei einer weiteren Ausführungsform der Anlage, kann es vorteilhaft sein, dass
die Reaktoreinheit einen weiteren Hauptreaktor als Festbettreaktor umfasst, der
   - einen ersten Strömungsweg für das Stoffgemisch und
   - einen weiteren (geschlossenen) Strömungsweg, d.h. einem. (Kühl-)Medienumlauf) für ein Wärmetauschmittel umfasst, und wobei stromaufwärts zu den beiden Hauptreaktoren in der (Zu-)Leitung eine Ventileinheit vorgesehen ist, mittels welcher der Volumenstrom der Eduktmischung zw. dem ersten Hauptreaktor und dem weiteren Hauptreaktor aufteilbar, durchleitbar und/oder vollständig umschaltbar ist, und wobei beide Hauptreaktoren
   - jeweils mit einem Wärmetauscher oder
   - einem gemeinsam mit einem Wärmetauscher
für den weiteren (geschlossenen) Strömungsweg verbunden sind.

Hierbei sind die beiden in Reihe geschalteten Hauptreaktoren baugleich oder im Wesentlichen baugleich. Insbesondere sind beide Hauptreaktoren derart dimensioniert und/oder weisen entsprechende Einbauten auf, dass dieselbe bzw. die im Wesentliche gleiche Masse und/oder Volumen an Katalysator angeordnet ist oder aufnehmbar ist.

Vorteil dieser beiden Hauptreaktoren liegt darin, dass eine weitere thermische Entkopplung der ersten Reaktionsphase mit sehr starker Exothermie, der zweite Reaktionsphase mit mittlerer Exothermie und der Nachreaktion mit sehr geringer Exothermie erfolgen kann. Weitere Vorteile bestehen darin, dass bei derselben Anlagenleistung, der einzelne Hauptreaktor kleiner dimensioniert ist und somit thermisch leichter und homogener geregelt werden kann. Zeitgleich wird die Anlageleistung erhöht, weil im Wartungsfall, wie bspw. einem Katalysatorwechsel, die Anlage nicht vollständig stillgestellt werden muss. Hierzu sind die beiden Hauptreaktoren derart leitungstechnisch verschaltet, dass jeder auch alleinig vom Stoffgemisch durchströmbar ist, unter Umgehung des jeweils andere Hauptreaktors (Bypass 1).

Durch die Hydrierung im (isothermischen) Hauptreaktor mit starker Kühlung, kann die durch die Temperatur induzierte Isomerisierung stark begrenzt werden. Im (adiabatischen) Nachreaktor wird anschließend gezielt so viel Temperatur insb. durch Wärmetausch im zufließenden Stoffstrom eingeregelt und damit zugelassen, dass die Isomerisierung gerade einen spezifikationsgerechten trans/trans-Anteil im Produkt liefert. Bei einer rein isothermen Betriebsweise eines einzelnen Hauptreaktors ohne Nachreaktor, würde man zunächst zu niedrige trans/trans-Anteile und einen hohen Anteil an nicht umgesetztem MDA erhalten. Durch die Möglichkeit den Stoffstrom gezielt adiabatisch hydrieren zu lassen, kann die Temperaturentwicklung und damit die Isomerisierung im Nachreaktor vorteilhaft gesteuert werden.

Bei einer weiter verbesserten Variante ist die leitungstechnische Verschaltung derart, dass auch der Nachreaktor beim Betrieb von mindestens einem Hauptreaktor umgangen entweder kann (Bypass 2), insb. aber beim Betrieb von den beiden in Reihe geschalteten Hauptreaktoren umgangen werden kann. Bei der Verschaltungsvariante Bypass 2, übernimmt der in Strömungsrichtung als zweiter durchflossene Hauptreaktor mind. zeitweise die Funktion des Nachreaktors, so dass die Anlage ohne bzw. weitgehend ohne Leistungsabfall und/oder Änderungen der Produktqualität, insb. des jeweiligen Anteils an Isomeren im Isomerengemisch betrieben werden kann.

Bei einer weiteren Ausführungsform der Anlage, kann es vorteilhaft sein, dass in der Leitung zwischen dem mind. einen Hauptreaktor und dem zweiten Hauptreaktor mind. ein gemeinsamer Wärmetauscher angeordnet ist. Dieser gemeinsame Wärmetauscher ist in einem zentralen Ast beider Kühlmittelumläufe angeordnet. Leitungsführung und Leitungsverschaltung ist dabei derart, dass nach dem gemeinsamen Wärmetauscher das Kühlmedium zuerst in den ersten der beiden Hauptreaktoren eingeleitet wird, in dem die stärker exotherme Reaktion abläuft. Anschließend wird über eine Leitung das bereits erwärmte Kühlmedium in den stromabwärts befindlichen zweiten Hauptreaktor geleitet, so dass dieser mit einer vom ersten Hauptreaktor unterschiedlichen Vorlauftemperatur beschickt wird.

Der Vorteil besteht darin, dass es überraschenderweise beobachtet wurde, dass insb. die sehr genaue Regelung der ersten, stark exothermen Reaktionsphase für die Produktqualität entscheidend ist, so dass auf den baulichen und regelungstechnischen Aufwand eines weiteren, vollständig unabhängigen zweiten Kühlkreislaufs verzichtet werden kann. Dies insb. auch deshalb, weil über die Regelung der Vorlauftemperatur des zu den beiden Hauptreaktoren seriell nachgeschalteten Nachreaktors die vollständige Reaktion sichergestellt und geregelt werden kann, insb. der gewünschte, niedrige trans/trans-Isomerenanteil.

Bei einer Anlagenvariante mit einer verbesserten Regelbarkeit kann vorgesehen werden, dass in der jeweiligen (Kreuz-)Leitung der Kühlkreisläufe, mit denen der Kühlmittelauslass des ersten Reaktors mit dem Kühlmitteleinlass des zweiten Reaktors verbunden ist, ein bedarfsweise schaltbarer und regelbarer Wärmetauscher (Nachkühler) angeordnet ist.

Der Sumpfumlauf des Trennkessels wird bei einer Temperatur von 130 °C bis 150 °C, idealerweise 135 bis 145 °C betrieben. Der große Vorteil des baulich und regelungstechnisch sehr einfachen Trennkessels ist, dass die Siedetemperatur des Gemisches durch die Druckabsenkung ebenfalls gesenkt wird, so dass nur auf diese erniedrigte Siedetemperatur im Trennkessel aufgeheizt werden muss. Weiterhin sind bereits durch diese Maßnahme ca. 90 % des in der Eduktmischung vorliegenden Lösungsmittels, insb. THF, abtrennbar. Ein Kopfkreislauf oder Rücklaufstrom, wie bei einer Kolonne, ist hierzu nicht erforderlich. Der zur ersten Trennkolonne weitergeleitete Stoffstrom weist somit vorteilhafterweise nur eine verbleibende Lösungsmittelkonzentration von ca. 20 bis 40 Gew.% auf, idealerweise 25 bis 35 Gew.%. Somit kann die erste Trennkolonne baulich kleiner ausfallen und ist aufgrund der geringeren Masse des Stoffstroms energetisch sparsamer betreibbar.

Auf diese Weise ist die Temperatur des Stoffstrom zudem schneller absenkbar, so dass im Stoffstrom ein Anstieg des Anteils an trans/trans-Isomeren des PACM verhindert bzw. verringert wird.

Die Lösungsmittelführende (Rück-)Leitung ist mit der Konditionierungseinheit und/oder mind. einem geeigneten Sammeltank verbunden.

Von der Erfindung ist weiterhin eine Verfahren zur kontinuierlichen, katalytischen Hydrierung von Methylendianilin (MDA; Edukt1) mit einem Wasserstoffgeber (Edukt2), insb. einem gasförmigen Wasserstoffgeber, vorzugsweises Wasserstoff (H2),
wobei die Herstellung mittels einer industriellen Anlage erfolgt, wobei die Anlage nach mindestens einem der hierin beschriebenen Ausführungsbeispielen und Varianten ausgebildet ist, und wobei der Hauptreaktor bei einer Temperatur im Bereich von 80 °C bis 150 °C betrieben wird, und wobei und wobei durch Dampfkompression im Zwischenkreislauf mittels des mind. einen Gebläses und/oder der Gebläsegruppe mind. eine Temperaturerhöhung des Mediums in dem hinführenden Leitungsast um 20 °C bis 60 °C vorgenommen wird, idealerweise von 30 °C bis 50 °C.

Bei einer weiteren Ausführungsform des Verfahrens kann ein weiterer Vorteil darin bestehen, dass der mind. eine Hauptreaktor bei einem Druck im Bereich von 60 bar bis 120 bar betrieben wird, idealerweise im Bereich von 70 bis 110 bar. Bei einer weiteren vorteilhaften Verfahrensführung kann vorgesehen werden, dass der Druck im Hauptreaktor 70 bis 100 bar beträgt, idealerweise 80 bis 90 bar beträgt. Besonders bevorzugt ist ein Druck von ca. 85 bis 90 bar.

Bei einer bevorzugten Ausführungsform des Verfahrens kann vorgesehen werden, dass eine kontinuierliche, katalytische Produktion von Methylenbis(cyclohexylamin) erfolgt, insb. die Produktion von 4,4'-Diaminodicyclohexylmethan (PACM), bevorzugt PACM mit niedrigen Anteilen an trans/trans-Isomeren. Bei einer bevorzugten Verfahrensvariante erfolgt eine kontinuierliche, katalytische Herstellung von Methylenbis(cyclohexylamin), insb. eine Produktion von PACM, nach der Formel (I)

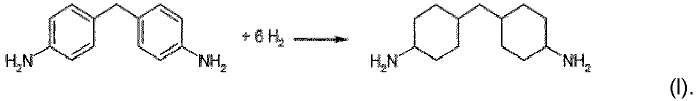

Bei einer weiteren Ausführungsform des Verfahrens kann ein weiterer Vorteil darin bestehen, dass neben dem mind. einen Hauptreaktor weiterhin mind. ein Katalysator enthaltender Nachreaktor umfasst ist, wobei der mind. eine Hauptreaktor und der mind. ein Nachreaktor bei demselben oder im Wesentlichen bei demselben Druck betrieben werden. Hierbei ist mit "im Wesentlichen" gemeint, dass der Druck in Haupt- und Nebenreaktor bis auf +/- 5 bar Unterschied auf dem gleichen Niveau ist. Insbesondere hat es sich als vorteilhaft herausgestellt, wenn der Druck im Nachreaktor geringfügig tiefer ist, als der im Hauptreaktor, insb. ca. 2 bar bis 5 bar niedriger. Der Katalysator des Nachreaktors ist ebenfalls eine immobiler Katalysator, der bspw. als Schüttung im Nachreaktor eingebracht ist. Vorteilhafterweise ist der Katalysator im Nachreaktor stofflich identisch oder weitgehend gleich zum Hauptreaktor, wobei die Form und/oder Einbringungsart des Katalysators im Nachreaktor unterschiedlich zum Hauptreaktor sein kann.

Bei einer weiteren Ausführungsform des Verfahrens kann ein weiterer Vorteil darin bestehen, dass vom Zeitpunkt t₀, dem Verfahrensstart nach Erneuerung oder Regeneration des Katalysators bis zum Zeitpunkt t₄, dem Ende des Verfahrens, bestimmt durch die Erneuerung oder Regeneration des Katalysators, die Betriebstemperatur des Hauptreaktors erhöht und Temperatur im Stoffstrom im Einlass (Feed) des Nachreaktors gleichgehalten oder gesenkt wird, wobei die Temperaturerhöhung oder -Absenkung linear und/oder schrittweise erfolgt.

Mit der genannten Anlagenvariante ist somit der folgende Verfahrensverlauf zu einer Erreichung eines niedrigen trans/trans-Anteils im PACM von 17 bis 25 Gew.% über der Zeit möglich.

Bei einer weiteren Ausführungsform des Verfahrens kann ein weiterer Vorteil darin bestehen, dass im Verteilerkreislauf eine mehrstufige Druckerhöhung in dem hinführenden Leitungsast erfolgt, wobei nach dem Verteilerkessel und vor dem ersten Verdichter ein Eingangsdruck von 1,5 bar bis 5 bar und eine Temperatur von 100°C bis 150°C vorliegt. Idealerweise ist der (Eingangs-)Druck 2 bar bis 4 bar, wobei eine Temperatur von idealerweise 120 bis 145°C vorliegt. Zusätzlich oder alternativ ist es vorteilhaft, wenn nach dem letzten Verdichter und vor dem ersten als Wärmesenke wirkenden Wärmetauscher in der hinführenden Leitung ein (End-)Druck von 10 bis 30 bar, idealerweise 15 bis 25 bar, und eine Temperatur von 180°C bis 300°C, idealerweise von 220 bis 280°C, vorliegen.

Bei einer weiteren Ausführungsform des Verfahrens kann ein weiterer Vorteil darin bestehen, dass im Verteilerkreislauf im ersten Leitungsast eine mehrstufige Druckerhöhung erfolgt, wobei nach dem letzten Verdichter und vor dem ersten als Wärmesenke wirkenden Wärmetauscher ein Druck von 3 bar bis 30 bar und eine Temperatur von 130°C bis 300°C vorliegt.

Bei einer weiteren Ausführungsform des Verfahrens kann ein weiterer Vorteil darin bestehen, dass die vom Sammelkreislauf in den ersten Verdampfer eingebrachte Energie mittels des Zwischenkreislaufs und dem mind. einen eingebundenen Gebläse und/oder der Gruppe aus x Gebläsen
- um mind. Faktor 1,1 bis 2,5 und/oder
- die Ausgangstemperatur des Kessels um 10 °C bis 80 °C angehoben wird, idealerweise um 20 °C bis 50 °C
angehoben wird.

Hierbei ist mit "Ausgangstemperatur" die Temperatur im Feed des Verteilerkessels, im Kopfauslass des Kessel und/oder und Sumpfauslass des Kessels gemeint. Gemäß einem ersten Ausführungsbeispiel ist der Verteilerkessel selbst nicht gesondert beheizt, abgesehen von der Erwärmung über das vom Zwischenkreislauf kommende Medium. In einer ersten Ausführungsform weist der Kessel selbst keine Heizeinheit oder einen innenliegenden Wärmetauscher auf und ist auch nicht an bspw. einen Sumpfkreislauf angeschlossen. Es kann eine Verbesserung darin bestehen, den Kessel mit einer gesonderten integrierten Heizung auszustatten oder an einen gesonderten Heizkreislauf anzuschließen, insb. um bedarfsweise ergänzend Energie zuzuführen.

Bei einer weiteren Ausführungsform des Verfahrens kann ein weiterer Vorteil darin bestehen, dass das MDA (Edukt1) eine Mischung der mind. zwei Isomere umfasst: 4,4' MDA, 2,4' MDA und 2,2' MDA, wobei der Anteil an 4,4' MDA vorteilhafterweise im Bereich von 75 bis 98 mol-% liegt, bevorzugt 85 bis 95 mol-%, idealerweise ca. 90 mol-%. Der Anteil an 2,4' MDA im Eduktgemisch beträgt vorteilhafterweise 7 bis 15 mol-%, bevorzugt 8 bis 12 mol-%, idealerweise 9 bis 10 mol-%.

Hierbei kann eine vorteilhafte Ausführungsform darin bestehen, dass das Lösungsmittel in einen Gewichtsanteil von 40 bis 50 Gew.% bezogen auf MDA in der Eduktmischung vorhanden ist.

Idealerweise ist der Anteil an trans/trans PACM im Bereich von 15 bis 30 Gew.%, idealerweise 16 bis 25 Gew.%.

Insgesamt ist es vorteilhaft, wenn der Reaktionsschritt im Hauptreaktor hierbei isotherm oder weitgehend isotherm und der Reaktionsschritt im Nachreaktor adiabat oder weitgehend adiabat betrieben wird. Zum Zeitpunkt t₀, dem Verfahrensstart kann neben dem Hauptreaktor auch der Katalysator im Nachreaktor erneuert oder regeneriert vorliegen. Vorteilhafterweise wird der Zyklus zur Regeneration oder Erneuerung des Nachreaktors eigenständig und vom Hauptreaktor unabhängig bestimmt, insb. wenn o.g. Zusammenwirken der Reaktionsanteile in beiden Reaktoren nicht mehr die Produktion des gewünschten niedrigen trans/trans-Anteils im PACM ermöglicht.

Bei einer vorteilhaften Ausführungsform des Verfahrens kann vorgesehen sein, dass die Temperatur des Eduktstroms am Einlass des Hauptreaktors 90 bis 140°C beträgt, idealerweise 95 bis 135 °C.

Bei einer weiteren vorteilhaften Ausführungsform des Verfahrens kann vorgesehen sein, dass der Druck im Hauptreaktor 70 bis 100 bar beträgt, idealerweise 80 bis 90 bar.

Bei einer weiteren vorteilhaften Ausführungsform des Verfahrens kann vorgesehen sein, dass - die Temperatur am Eingang des Hauptreaktors im Wesentlichen der Temperatur am Eingang des Nachreaktors entspricht, wobei im Wesentlichen einen Bereich oder Unterschied von +/- 10 °C meint und/oder
der Druck am Eingang des Hauptreaktors im Wesentlichen dem Druck am Eingang des Nachreaktors entspricht, wobei im Wesentlichen einen Bereich oder Unterschied von +/- 5 bar meint.

Insgesamt sollen alle Aspekte, Vorteile und Ausführungen zur Anlagen oder in Zusammenhang mit deren Beschreibung genannt sind, identisch oder in analoger Weise auch für das Verfahren gelten und umgekehrt, sofern nicht etwas Abweichendes dargelegt ist und/oder eine technische Unmöglichkeit der analogen Anwendung gegeben ist.

Nachfolgend wird die erfindungsgemäße Lösung anhand von Ausführungsbeispielen im Detail beschrieben. Es zeigen:
- Fig. 1: eine Anlage als Prozessfließbild,
- Fig. 2: eine erste Ausführungsform der drei Medienkreisläufe,
- Fig. 3: eine zweite Ausführungsform der Medienkreisläufe mit einer Option zur Direktverschaltung einer Gruppe von Wärmetauschern,
- Fig. 4: eine dritte Ausführungsform der Medienkreisläufe mit einer Option zur Direktverschaltung einer Gruppe von Wärmetauschern,
- Fig. 5: eine Ausführungsform der Reaktoreinheit,
- Fig. 6: eine weitere Ausführungsform der Reaktoreinheit
- Fig. 7: eine vierte Ausführungsform der Medienkreisläufe mit einem weiteren Verteilerkessel zwischen Sammelkreislauf und Zwischenkreislauf und
- Fig. 8: eine fünfte Ausführungsform der Medienkreisläufe, als Variante der Ausführungsform der Figur 7.

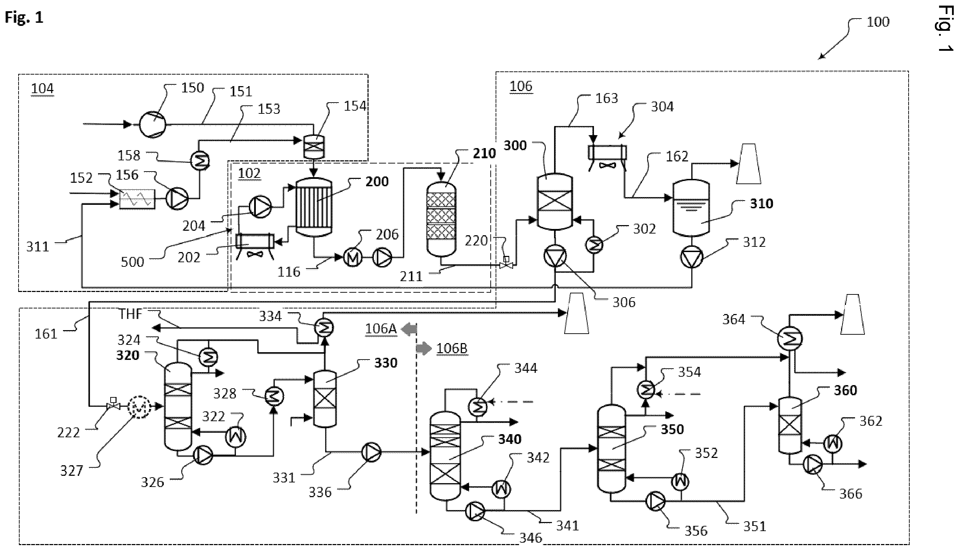

Die Figur 1 zeigt die Anlage 100 zur kontinuierlichen Herstellung von 4,4'-Diaminodicyclohexylmethan (PACM) durch katalytische Hydrierung von Methylendianilin (MDA; Edukt1), insb. 4,4`-Diaminodiphenylmethan, mit einem Wasserstoffgeber (Edukt2), wobei der Wasserstoffgeber als gasförmiger Wasserstoff (H2) zugeführt wird. Die Anlage 100 umfasst eine Konditionierungseinheit 104 für die Edukte, eine Reaktoreinheit 102 und eine Trenneinheit 106.

Die Konditionierungseinheit 104 ist gestrichelt umrahmt und umfasst (Zu-)Leitungen für das Edukt1 und den Wasserstoff (Edukt2) sowie das Lösungsmittel. Weiterhin umfasst die Konditionierungseinheit eine Verdichtereinheit 150, nachfolgend Verdichter genannt in der den Wasserstoff zuleitenden Leitung 151, einen Mischer 152 in der das Lösungsmittel und das Edukt1 zuführenden Leitung. Zudem sind in der die Mischung aus Edukt1 und Lösungsmittel zuführenden Leitung 153 eine Pumpe 156 und ein Wärmetauscher 158 angeordnet. Die Leitungen 151, 152 münden in einen Mischbehälter 154, der stromaufwärts zum Hauptreaktor 200 angeordnet ist. Der Mischbehälter 154 dient der intensiven Vermischung der Edukte und dessen Auslauf bildet den Zulauf für den Hauptreaktor 200.

Die Reaktoreinheit 102 ist gestrichelt umrahmt und umfasst im Wesentlichen den Hauptreaktor 200, einen Kühlumlauf 500, eine Nachreaktor 210 und einen Wärmetauscher 206 in der Zulaufleitung zum Nachreaktor 210. In den Kühlumlauf 500 ist eine Pumpe 204 und ein Wärmetauscher 202 eingebunden, wobei das umlaufende Kühlmittel im Hauptreaktor 200 die mit Katalysatormaterial befüllten Trägerelemente umströmt. Im gezeigten Beispiel werden die mit Katalysatormaterial befüllten Trägerelemente im Gleichstromrichtung angeströmt. Der Nachreaktor 210 ist über die Leitung 211 mit dem Trenneinheit 106, bzw. deren ersten Trennstufe verbunden.

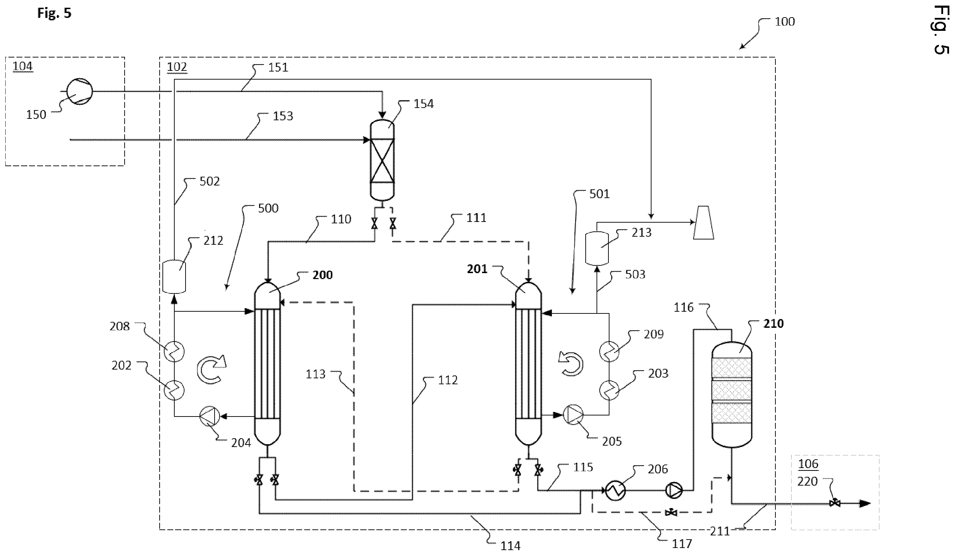

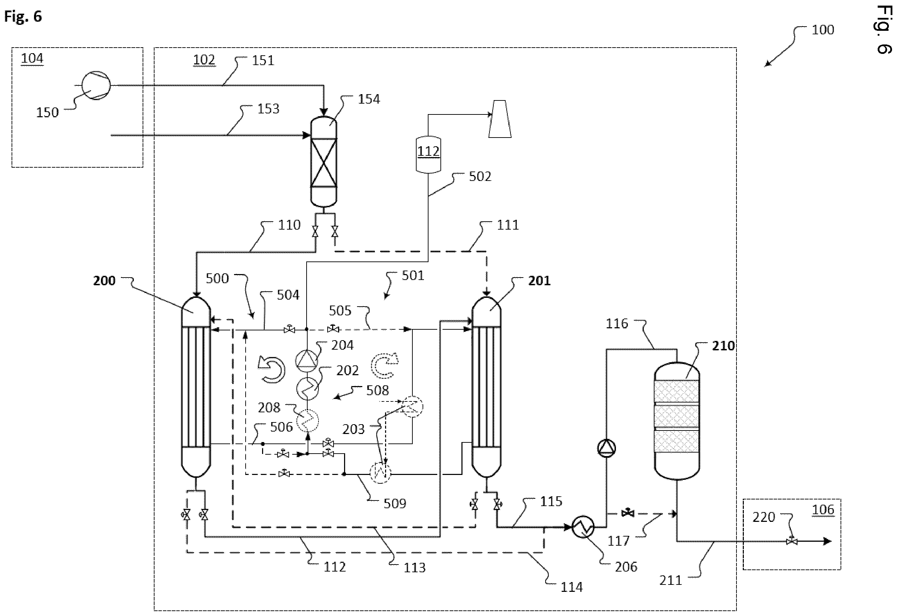

Der Wärmetauscher 202 im Kühlumlauf 500 ist als luftgekühlter Wärmetauscher 202 dargestellt, kann aber auch alternativ ausgebildet sein, um bspw. mittels eines fließenden Kühlmediums, wie bspw. ein Öl, Wasser oder eine Sole, um im indirekten Wärmeaustausch das Kühlmedium des Kühlumlaufs 500 zu temperieren. In den Kühlumlauf 500 ist weiterhin bei einer nichtdargestellten Anlagenvariante ein Wärmetauscher eingebunden, analog den Wärmetauschern 208, 209 der Figuren 5, 6. Dieser wird mit einem Heizmedium betrieben und dient im Anfahrschritt des Hauptreaktors 200 zur Temperierung des Hauptreaktors 200 auf ca. 80 bis 100 °C, idealerweise auf eine Temperatur von 85 °C bis 95 °C. In dem gezeigten Anlagen- und Verfahrensbeispiel, bei dem Ziel ein möglichst niedriges trans/trans-Isomerenverhältnis von ca. 17 bis 23 Gew.% zur realisieren, wird der mit frischem oder regeneriertem Katalysator befüllte Hauptreaktor 200 auf eine Temperatur von ca. 90 °C mittels des Wärmetauschers 208 vorgeheizt. Der Hauptreaktor 200 wird bei einem Druck von 87 bis 88 bar betrieben.

Die Trenneinheit 106 ist gestrichelt umrahmt und umfasst eine Mehrzahl von Trennapparaten zur Abtrennung des Lösungsmittel insb. in einer ersten Trennstufe und des Produkts PACM, insb. in einer zweiten Trennstufe, von restlichem Edukt und Nebenprodukten. Die erste Trennstufe (nicht ausgewiesen) umfasst einen Trennkessel 300 an den ein Sumpfumlauf angeschlossen ist, in den ein Wärmetauscher 302 und eine Pumpe 306 eingebunden ist. Der Kopfauslass des Trennkessels 300 ist mit einem Wärmetauscher 304, einem Kondensator, verbunden, zu dem stromabwärts ein Sammelbehälter 310 für das Lösungsmittel vorgesehen ist. Mittels des Wärmetauschers 304 (Kondensator) werden ca. 80% des Lösungsmittels, vorliegend THF, auskondensiert und könnten gesammelt bzw. zurückgeleitet werden. In der Flash-Stufe ist ein Energiebedarf für den Wärmetauscher 302 im Sumpfumlauf des Trennkessels 300 von ca. 1400 kW erforderlich.

Der Kondensator 304 ist als Wärmetauscher in der Bauform eines luftgekühlten Apparates dargestellt, kann aber auch alternativ ausgebildet sein, um bspw. mittels eines fließenden Kühlmediums, wie bspw. Kühlwasser oder einem zur Wärmeintegration geeigneten Medium, um im indirekten Wärmeaustausch den eingangs dampfförmigen Stoffstrom mind. teilweise zu kondensieren und zu kühlen.

Die hierin genannten Energiemengen sind kalkuliert für eine Anlagenleistung des Produktes PACM bei der genannten Synthesereaktion von ca. 3,37 t/h, sowie eine Nebenproduktleistung von ca. 0,4 t/h an HB und ca. 0,05 t/h an LB. Das Verhältnis der Massenströme von THF zu MDA betrugt 4,2 bis 4,5.

Weiterhin umfasst die erste Trennstufe der Trenneinheit 106 zur weitergehenden Abtrennung des Lösungsmittels einen erste Trennkolonne 320 und eine zweite Trennkolonne 330, wobei die zweite Trennkolonne 330 als Stripping Kolonne ausgebildet ist. Hierzu wird vorteilhafterweise Stickstoff (N2) als Stripping-Medium eingesetzt, das im Gegenstrom zum Stoffstrom durch die Kolonne geleitet wird. Mittels der im Sumpfauslass des Trennkessels 300 befindlichen Pumpe 306 ist der an Lösungsmittel verarmte Stoffstrom über die Leitung 161 zur ersten Trennkolonne 320 ableitbar. In der Leitung 161 ist eine Druckregeleinheit 222 vorgesehen, die im gezeigten Beispiel als regelbares Ventil ausgebildet ist. Der ersten Trennkolonne 320 wird der Stoffstrom über einen zentralen Zulauf wie dargestellt eingeleitet. Weiterhin ist stromaufwärts zur ersten Trennkolonne 320 ein (Vorlauf-)Wärmetauscher 327 (gestrichelt dargestellt) angeordnet, der eine Option zur Beheizung der ersten Trennkolonne 320 darstellt.

In der ersten Trennkolonne 320 wird die Lösungsmittelkonzentration von 30 Gew.% im Zulauf über den produktreichen Stoffstrom der Leitung 161 auf ca. 2 Gew.% an restlichem Lösungsmittel verringert.

Diese erste, mit (Struktur-)Packungen bestückte, Trennkolonne 320 ist an einen Sumpfumlauf angeschlossen, in den ein Wärmetauscher 322 und eine Pumpe 326 eingebunden sind. Weiterhin ist am Kolonnenkopf der ersten Trennkolonne 320 ein Kopfumlauf angeordnet, in den ein Wärmetauscher 324 eingebunden ist, der als Kondensator ausgebildet ist. Aus dem Kopfumlauf führen zwei Ableitungen für den lösungsmittelreichen Stoffstrom, wobei eine der beiden Ableitungen in die Kopfableitung der stromabwärts befindlichen Trennkolonne 330 (Stripping-Kolonne) einmündet.

Insbesondere ist das in der ersten Trennstufe abgetrennte Lösungsmittel über die Leitung 311 in einen Sammeltank (nicht dargestellt) und/oder den Mischer 152 der Konditionierungseinheit 104 leitbar. In der vom Nachreaktor 210 zum Trennkessel 300 führenden Leitung 211 ist eine Druckregeleinheit 220 vorgehen, die im vorliegenden Beispiel als steuerbares Ventil ausgebildet ist.

Über die Leitung 321 wird aus dem Sumpfauslass der ersten Trennkolonne 320 der produktreiche Stoffstrom mit einem Restanteil von ca. 2 Gew.% Lösungsmittel (THF) in den Kopf der zweiten Kolonne 330, der Stripping-Kolonne, geleitet. In dieser Leitung 321 ist ein Wärmetauscher 328 eingebunden. Die zweite Kolonne 330 weist mind. eine innere Packung auf, wobei unterhalb der Packung eine Gaszuleitung für insb. ein Inertgas (Strippinggas) angeordnet ist, so dass der eingeleitete produktreiche Stoffstrom im Gegenstromprinzip zum Strippinggas die zweite Kolonne 330 durchströmt und von Lösungsmittel weiter abgereichert wird. In dem gezeigten Anlagenbeispiel wird Stickstoff (N2) als Strippinggas eingesetzt. Der lösungsmittelreiche Dampf wird über den Kopfauslass in einen Kondensator 334 geleitet und gemeinsam mit dem von Kopfauslass der ersten Trennkolonne 320 kommenden und die Kopfableitung der zweiten Trennkolonne 330 eingeleitete Dampfstrom dem Kondensator 334 zugeleitet. Der im Wärmetauscher 334 auskondensierte Lösungsmittelstrom wird zur Konditionierungseinheit 104 zurückgeleitet, wobei der nicht-kondensierbare Anteil aus dem Wärmetauscher 334 abgeleitet und bspw. vollständig thermisch oxidiert wird.

Vom Sumpfauslass der zweiten Trennkolonne 330 wird der produktreiche Stoffstrom über die Leitung 331, in die die Pumpe 336 eingebunden ist, der dritten Trennkolonne 340 zugeführt. Die zweite Trennstufe 106B der Trenneinheit 106 dient insb. der Abtrennung der Nebenprodukte LB und/oder HB vom Produkt PACM.

Die zweite Trennstufe umfasst im Wesentlichen drei Trennkolonnen, wobei der dritten Trennkolonne 340, die erste der zweiten Trennstufe, der Stoffstrom zentral zugeführt wird. Die dritte Trennkolonne ist mit einem Sumpfumlauf verbunden, in den ein Wärmetauscher 342 und eine Pumpe 346 eingebunden sind. Der produktreiche Stoffstrom wird über die Leitung 341 aus dem Sumpfablauf zur vierten Trennkolonne 350 geleitet. Weiterhin ist die dritte Trennkolonne 340 mit einem Kopfumlauf verbunden, in den ein Wärmetauscher 344 eingebunden ist. Aus diesem Kopfumlauf wird auskondensiertes LB als erstes Nebenprodukt abgeleitet.

Der produktreiche Stoffstrom wird über die Leitung 341 der vierten Trennkolonne 350 zentral zugeführt. Die vierte Trennkolonne 350 ist mit einem Sumpfumlauf verbunden, in den ein Wärmetauscher 352 und eine Pumpe 356 eingebunden sind. Weiterhin ist die vierte Trennkolonne 350 mit einem Kopfumlauf verbunden, in den ein als Kondensator ausgebildeter Wärmetauscher 354 eingebunden ist. Der produktreiche Stoffstrom wird über die Kopfableitung aus dem Kopfumlauf als Kondensat ausgeleitet. Vorliegend wird gemäß dem gezeigten Beispiel, der nichtkondensierte Dampf- und/oder Gasstrom in die Kopfausleitung der stromabwärtsbefindlichen fünften Trennkolonne 360 eingeleitet. Nachfolgend wird über einen weiteren Wärmetauscher 364 (Kondensator) Produkt weiter auskondensiert und ausgeleitet. Der produktarme Stoffstrom wird über die Sumpfableitung der vierten Trennkolonne 350 über die Leitung 351 abgeleitet und der fünften Trennkolonne 360 in deren Kopfteil eingeleitet. Dieser Stoffstrom ist stark angereichert an HB, einem zweiten Nebenprodukt. Die fünfte Trennkolonne 360 ist mit einem Sumpfumlauf verbunden, in den ein Wärmetauscher 362 und eine Pumpe 366 eingebunden sind. Weiterhin weist die Trennkolonne 360 einen Kopfauslass auf, der zu dem vorgenannten als Kondensator ausgebildeten Wärmetauscher 364 führt. In diesem Kondensator 364 wird ein weiterer produktreicher Stoffstrom als Kondensat auskondensiert und ausgeleitet, wobei der nicht-kondensierbare Anteil gasförmig ausgeleitet wird. Dieser kann nachfolgend insb. vollständig oxidiert werden.

Die Reaktoreinheit 102 wird bzgl. des Stoffstroms auf einem ersten, hohen Druckniveau von ca. 60 bis 120 bar betrieben, die erste Trennstufe 106A der Trenneinheit 106 wird bei einem zweiten, niedrigen Druckniveau von 4 bis 12 bar betrieben und die erste Trennkolonne 320 und die zweite Trennkolonne 330 werden auf einem dritten, geringfügig erhöhten Druckniveau von 1,05 bis 2,5 bar betrieben. In dem gezeigten Beispiel ist das erste Druckniveau 80 bis 90 bar, das zweite Druckniveau 4,5 bis 7,5 bar und das dritte Druckniveau 1,1 bis 1,2 bar.

Mit der direkten Temperaturregelung des Hauptreaktors 200 über den Kühlkreislauf 500 in Reihe mit dem ungekühlten Nachreaktor 210, hat sich überraschenderweise eine gegenüber dem Stand der Technik verringerter Energiebedarf und eine vereinfachte, stabilere Verfahrensführung gezeigt.

Ohne auf eine spezielle Interpretation gebunden zu sein, wird dieser Erfolg darin gesehen, dass der Kühlkreislauf 500 nur gezielt für die sehr heftige Anfangsreaktion zur Ableitung der exothermen Reaktionswärme ausgelegt werden muss, wobei die noch bedeutsame Nachreaktion alleinig über die Zulauftemperatur mittels des stromaufwärts befindlichen Wärmetauscher 206 eingestellt werden muss. Der Stoffstrom wird durch die bereits stark abgeschwächte Reaktion im Nachreaktor 210 nur noch um ca. 5 bis 15 °C erwärmt und kann in diesem Niveau unproblematisch in den Trennkessel 300 entlassen werden.

Insgesamt sind in den Figuren in den Apparaten, wie Reaktoren, Trennkolonnen, Behältern etc., über die entsprechenden Symbole Einbauten, wie Packungen, Trennebenen, Trägerelemente etc. skizziert. Diese deuten jeweils vorteilhafte Ausführungsformen an, betreffend die Anzahl, Art und/oder relative Lage zur jeweils zu- oder abführenden Leitung. So ist bspw. mit der Darstellung ersten Trennkolonne 320 angedeutet, dass vorteilhafterweise der (Feed-)Stoffstrom über die Leitung 161 derart eingeleitet wird, dass zwischen dem Kopfumlauf und dem Sumpfumlauf jeweils mind. eine (theoretische) Trennebene vorhanden ist. Die Ermittlung der konkreten Art und/oder Anzahl von Trennebenen ist dem Fachmann bekannt und kann in geeigneter Weise variiert bzw. vorgesehen werden.

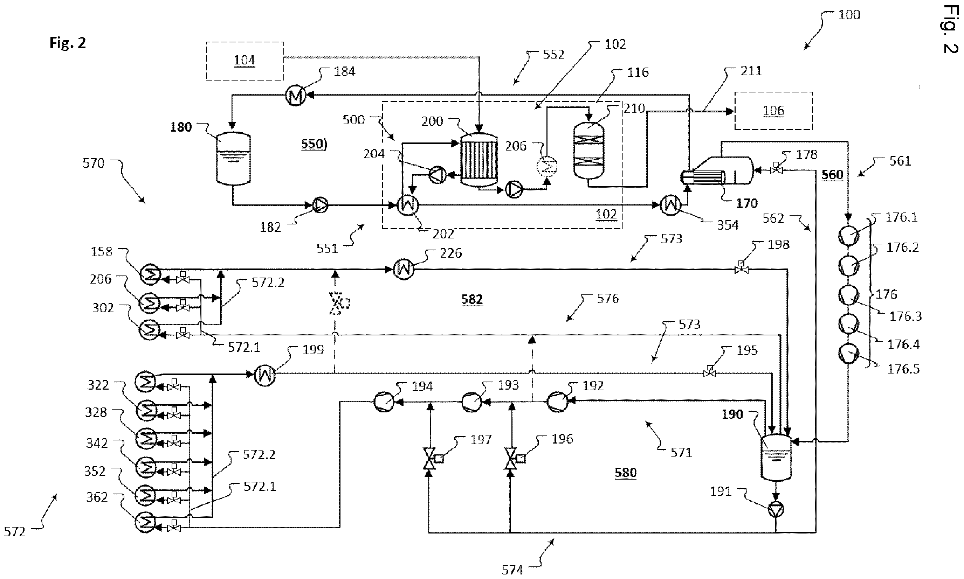

Figur 2 zeigt eine erste Ausführungsform einer Energieverschaltung, wobei drei Kreisläufe vorgesehen sind, nämlich ein Sammelkreislauf 550, ein Zwischenkreislauf 560 und ein Verteilkreislauf 570. Der Sammelkreislauf 550 umfasst einen Sammeltank 180, eine erste Leitung 551, einen Verdampfer 170 und eine zweite Leitung 552, wobei die erste Leitung 551 den Sammeltank 180 mit einem Einlass eines Wärmetauscher-Teils (WT-Teil) des Verdampfers 170 verbindet und die zweite Leitung 552 einen Auslass des WT-Teils des Verdampfers 170 mit dem Sammeltank 180 verbindet. Es ist somit nur das WT-Teil des Verdampfers 170 Teil des Sammelkreislaufs 550.

In die erste Leitung 551 sind der Wärmetauscher 202 des Kühlmittelumlaufs 500 des Hauptreaktors 200 und der Wärmetauscher 354 aus dem Kopfumlauf der vierten Trennkolonne 350 als Wärmequellen eingebunden, sowie eine Pumpe 182. In der zweiten Leitung 552 ist in dem gezeigten Beispiel im Vorlauf zum Sammeltank 180 ein Wärmetauscher 182 eingebunden, der im Wesentlichen dazu dient, bedarfsweise und aus Regelungsaspekten die erforderliche Zulauftemperatur (Kühlung) des Sammeltanks 180 und/oder die Temperatur in der ersten Leitung 551 einzustellen, um den Temperaturgradienten für die erforderliche Kühlung der eingebundenen Wärmetauscher 202, 354 zu gewährleisten. Die erste Leitung 551 kann auch als Sammel- oder Feedleitung betrachtet werden und die zweite Leitung 552 als Rückleitung. Als im Kreislauf strömendes Medium ist in dem gezeigten Beispiel Wasser vorgesehen, wobei auch eine Sole oder ein Öl genutzt werden könnte.

Durch den Sammelkreislauf werden auf vorteilhafter Weise mehrere Wärmequellen "eingesammelt" und derart gesammelt die Energiemenge des im Kreislauf geführten Mediums, hier insb. Wasser, an den ersten Verdampfer zu übertragen. Dies verringert den konstruktiven Aufwand erheblich, verglichen mit einer direkten Verschaltung der betroffenen Wärmetauscher.

Der Zwischenkreis 560 umfasst den vorstehend genannten Verdampfer 170, eine erste (hinführende) Leitung 561, eine Gebläsegruppe 176, eine rückführende Leitung 562 und eine Druckregeleinheit 178. Von dem Verdampfer 170 ist der Kesselteil (K-Teil) Teil des Zwischenkreislaufes 560, in den auch der Verteilerkessel 190 offen eingebunden ist. D.h. vom Wärmetauschmedium des Zwischenkreislaufs 560 wird auch der Verteilerkessel 190 und der Verteilerkreislauf 570 durchflossen. Als Wärmetauschmedium dient vorteilhafterweise Wasser bzw. Wasserdampf. Stromaufwärts zum Einlass der rückführenden Leitung 562 in den K-Teil des Verdampfers 170 ist die Druckregeleinheit 178 angeordnet. Die Gebläsegruppe 176 umfasst in dem gezeigten Beispiel fünf Gebläse 176.1 ... 176.5, die in der zuführenden Leitung 561 eingebunden und zueinander in Serie geschaltet sind.

In der ersten, hinführenden Leitung wird der Wasserdampf geführt und in den fünf Kompressionsstufen von einem Anfangsdruck nach dem Verdampfer 170 von ca. 90 °C auf ca. 135 °C überhitzt. Der Druck wird durch die Gebläsegruppe 176 von ca. 0,5 bis 1 bar auf ca. 3 bar angehoben. Der Medienstrom, insb. der Dampfanteil, wird im Verteilerkessel 190 aufgenommen, im Verteilerkreislauf 570 zirkuliert und dort durch Wärmeabgabe an die als Wärmesenke arbeitenden Wärmetauscher abgekühlt, so dass der Wasserdampf mind. teilweise auskondensiert und an den Verteilerkessel 190 zurückfließt. Über die (Sumpf-)Pumpe 191 und die rückführende Leitung 562 und die Druckregeleinheit 178 wird das Medium zum WT-Teil des Verdampfers 170 zurückgeleitet und erneut verdampft.

Neben der Möglichkeit das Temperaturniveau bedeutsam anzuheben, besteht ein weiterer Vorteil des Zwischenkreislaufs darin, dass als Wärmetauschmedium im Zwischenkreislauf und im Verteilerkreislauf Wasser bzw. Wasserdampf nutzbar ist. Die Nutzung von Wasser/-dampf in den gekoppelten beiden Kreisläufen hat den Vorteil, dass Wasser/-dampf bereits in der Anlage 100 als Wärmetauschmedium vorhanden ist, so dass keine zusätzlichen Risiken bzgl. Produktkontamination aufgrund Leckagen entstehen.

Grundsätzlich könnte auch ein anderes geeignetes Wärmetauschmedium genutzt werden, wie insb. ein Alkohol, insb. ein Alkohol mit 1 bis 6 Kohlenstoffatomen (C1-6 Alkohol). Dies kann bspw. Methanol, Ethanol, Isopropanol, Propanol, (Tert-, Iso-)Butanol sein.

An den Verteilerkessel 190, der in den Zwischenkreislauf 560 eingebunden ist, schließt sich der Verteilerkreislauf 570 an, der einen HD-Verteilerkreislauf 580 und einen ND-Verteilerkreislauf 582 umfasst. Der HD-Verteilerkreislauf 580 umfasst einen ersten, hinführenden Leitungsast 571, einen zweiten, verteilenden Leitungsast oder Leitungsabschnitt 572 und einen dritten, rückführenden Leitungsast 573, auch (Rück-)Leitung genannt. Ein Kopfauslass des Verteilerkessels 190 führt in den ersten Leitungsast 571, wobei im gezeigten Ausführungsbeispiel drei in Reihe geschaltete Verdichter 192, 193, 194 in den ersten Leitungsast 571 eingebunden sind. Weiterhin umfasst der HD-Verteilerkreislauf 580 eine (Sumpf-)Leitung 574, die von einem Sumpfauslass des Verteilerkessels 190 über zwei Leitungsknoten (Zwischeneinspeisung) in den ersten Leitungsast 571 mündet, wobei eine Pumpe 191 in der (Sumpf-)Leitung 574 eingebunden ist. Die erste Einleitung von Heizmedium erfolgt über den ersten Leitungsknoten zwischen dem ersten Verdichter 192 und dem zweiten Verdichter 193, die zweite Einleitung von Heizmedium erfolgt zwischen dem zweiten Verdichter 193 und dem dritten Verdichter 194. Stromaufwärts zu den jeweiligen, nicht bezeichneten Leitungsknoten ist jeweils ein regelbares Ventil 196, 197 angeordnet.

An den Verteilerkessel 190 schließt sich weiterhin der Verteilerkreislauf 570 mit einem zum HD-Verteilerkreislauf 580 parallelen ND-Verteilerkreislauf 582 an. Der ND-Verteilerkreislauf 582 umfasst ebenfalls einen ersten, hinführenden Leitungsast 576, einen zweiten, verteilenden Leitungsast oder Leitungsabschnitt 572 und einen dritten, rückführenden Leitungsast 573, auch (Rück-)Leitung genannt. In den hinführenden Leitungsast 576 ist in dem gezeigten Beispiel kein Verdichter eingebunden, so dass der Wasserdampf vom Verteilerkessel 190 mit ca. 3 bar und bei einer Temperatur von ca. 133 °C zu den als Wärmesenke fungierenden Wärmetauschern 158, 206, 302 geleitet wird. In der rückführenden Leitung 573 ist eine Druckregeleinheit 198 angeordnet, worüber die Entspannung auf den im Verteilerkessel 190 bestehenden Druckniveaus bzw. der erforderliche Druckgradient im ND-Verteilerkreislauf 582 geregelt wird.

In dem verteilenden Leitungsabschnitt 572 sind als Wärmesenken zu versorgende Wärmetauscher eingebunden, dies sind die zueinander parallel geschalteten Wärmetauscher 322, 328, 342, 352 und 362, die alle Wärmetauscher aus Sumpfkreisläufen der Trennkolonnen sind. In dem parallelen verteilenden Leitungsabschnitt 572 des ND-Verteilerkreislaufs 582 sind als Wärmesenken weitere zu versorgende Wärmetauscher eingebunden, dies sind die zueinander parallel geschalteten Wärmetauscher 158, 206, 302.

Eine zentrale Verteilleitung 572.1 führt zu den jeweiligen Wärmetauschern einer Gruppe und eine zentrale Sammelleitung 572.2 wird von den jeweiligen Wärmetauschern einer Gruppe gespeist und führt in die jeweilige (Rück-)Leitung 573 des HD- oder ND-Verteilerkreislaufs. Der verteilende Leitungsabschnitt 572 ist über die (Rück-)Leitung 573 mit dem Verteilerkessels 190 verbunden. In diese (Rück-)Leitungen 573 sind jeweils ein Wärmetauscher 199, 226 und eine Druckregeleinheit 195, 198 eingebunden, worüber der Enddruck des dritten Verdichters 194 von 20 bar bzw. der Dampfdruck des ND-Verteilerkreislaufs wieder auf das Niveau des Verteilerkessel von ca. 2,5 bis 3,5 bar entspannt wird. Die (Rück-)Leitungen 573 sind jeweils mit einem Einlass des Verteilerkessels 190 verbunden.

Der gezeigte Verdampfer 170 ist ein so genannter Kettle-Type-Verdampfer, der einen für ein erstes strömendes Medium geschlossenen Wärmetauscher-Teil (WT-Teil) und ein für ein weiteres strömendes Medium offenen Kessel-Teil (K-Teil) aufweist. Der WT-Teil weist einen Einlass und einen Auslass auf, wobei das Wärmetauschmedium in geschlossenen Kanälen oder Rohren geführt wird, wie bspw. mind. einem Rohrbündel. Der K-Teil weist mind. einen Einlass und jeweils einen (Kopf- oder Dampf-)Auslass auf, wobei der Verteilerkessel 190 auch einen (Sumpf-)Auslass aufweisen kann. Eingeleitetes flüssiges Medium, hier über die rückführende Leitung 562, wird mittels des WT-Teils bzw. des zugehörigen Wärmetauschers erwärmt und mind. teilweise verdampft. Der K-Teil kann zweiteilig sein oder zwei Teilräume aufweisen. Hierbei ist ein zentraler K-Teil vorhanden, in welchen auch der Wärmetauscher des WT-Teils hineinragt und der Energieeintrag in den K-Teil erfolgt. Der weitere Teilraum ist in einem seitlichen oder äußeren K-Teil angeordnet. Dieser kann vorteilhafterweise, aber nicht zwingend, durch Einbauten als eine für das flüssige Medium beruhigte Zone ausgebildet sein und/oder ist dadurch bestimmt, dass der Wärmetauscher des WT-Teils in diesen Teilraum nicht hineinragt.

Der wasserführende Verteilerkreislauf weist am hinführenden Leitungsast 571 des HD-Verteilerkreislaufs 580 unmittelbar stromabwärts zum Verteilerkessel 190 eine Temperatur von 133 °C und 3 bar auf. Nach dem ersten Verdichter 192 beträgt der Druck 6 bar, der mit einem Energieverbrauch von 71 kW erreicht wird, nach dem zweiten Verdichter 193 beträgt der Druck 12 bar, der mit einem elektrischen Energieverbrauch von weiteren 80 kW erreicht wird und nach dem dritten Verdichter 194 beträgt der Druck 20 bar bei einer Temperatur von 250 °C, erreicht durch weiteren elektrischen Energieverbrauch von 71 kW für den dritten Verdichter 194.

Zur Versorgung der in der Figur 2 gezeigten Wärmetauscher des HD-Verteilerkreislaufs 580, die insb. die Sumpfwärmetauscher der Trennkolonnen sind, muss ein sehr hohes Temperaturniveau bereitgestellt werden, so dass über den Bedarfs- und Regelwärmetauscher 199 das zirkulierende Medium wieder gekühlt werden muss. Die erforderliche Kühlleistung beträgt im gezeigten Beispiel ca. - 20 kW, die in der (Rück-)Leitung 573 vor der Druckregelungseinheit 195 bzw. vor Eintritt in den Verteilerkessel 190 abgeführt werden muss.

In der Figur 2 sind zwei weitere Ausführungsvarianten gezeigt, die als gestrichelte Leitungen skizziert sind. Gemäß der ersten Variante zweigt der ND-Verteilerkreislauf 582 nicht vom Verteilerkessel 190, sondern stromabwärts zum ersten Verdichter 192 ab, wird also auf dem erhöhten, ersten Druckniveau betrieben. Die zweite Variante, die mit der ersten kombinierbar ist, wird rückführende Leitung 573 stromabwärts zu einer Druckregeleinheit (auch gestrichelt dargestellt) in die rückführende Leitung 573 des ND-Verteilerkreislaufs 582 eingeleitet und auf diesem Weg zum Verteilerkessel 190 geleitet. Hierbei kann vorteilhafterweise auf einen der Sicherheit- und Bedarfswärmetauscher 199, 226 in den rückführenden Leitung 573 verzichtet werden.

Ein bedeutsamer Vorteil des Verteilerkreislaufes kann darin gesehen werden, dass ein zentraler Dampfkreislauf geschaffen wurde und somit Dampf zentral erzeugt und auf alle als Verbraucher (Wärmesenke) arbeitenden Wärmetauscher verteilt werden kann, anstelle der direkten Verschaltung von Wärmequellen und Wärmesenken. Weiterhin kann der Verteilerkreislauf als zentraler Dampfkreislauf im Bedarfsfall (z.B. beim Anfahren der Anlage) mindestens zeitweise mittels einer alternativen Wärme- oder Dampfquelle gespeist werden.

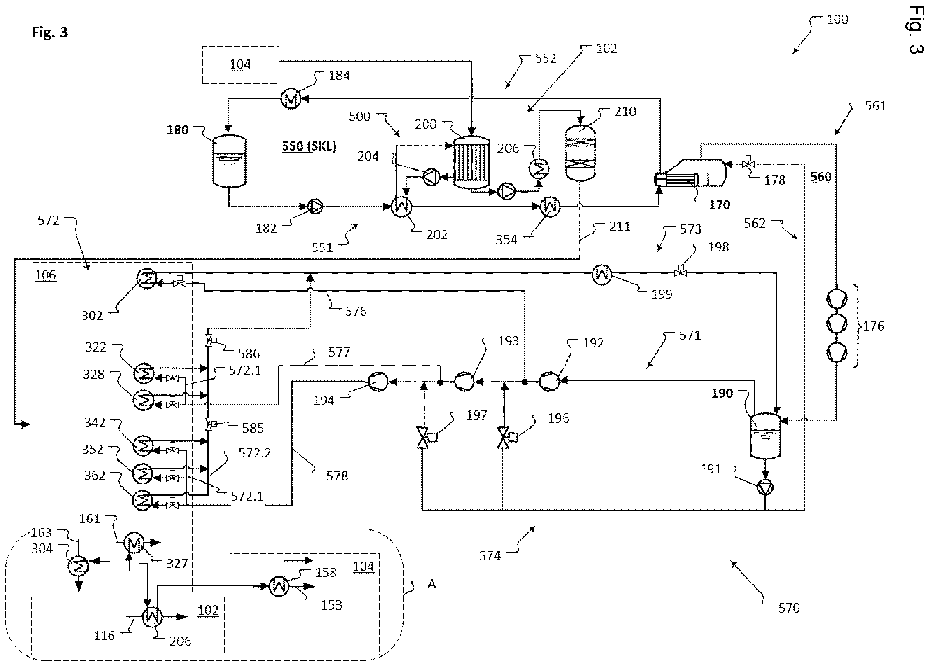

Figur 3 zeigt eine Ausführungsform, in der der Sammelkreislauf 550 und der Zwischenkreislauf 560 analog ausgebildet sind zur Figur 2. Der Verteilerkreislauf 570 unterscheidet sich zur Ausführungsform nach Figur 2 dadurch, dass der Leitungsabschnitt 572, über den die Energieverteilung an die als Wärmesenken arbeitende Wärmetauscher erfolgt, in den des HD-Verteilerkreislaufs 580 und zwei Teilabschnitte des ND-Verteilerkreislaufs 582 aufgeteilt ist, wobei diese drei Teilabschnitte zueinander parallel geschaltet sind:
- der HD-Verteilerabschnitt, angeschlossen stromabwärts zum dritten Verdichter 194,
- ein Mitteldruck-Teilabschnitt (MD-Teilabschnitt) des ND-Verteilerkreislaufs 582, angeschlossen stromabwärts zum zweiten Verdichter 193 und
- ein Niederdruck-Teilabschnitt (ND-Teilabschnitt) des ND-Verteilerkreislaufs 582, angeschlossen
stromabwärts zum ersten Verdichter 192.

Jeder Teilabschnitt weist eine eigene Verteilleitung 572.1 auf und ist über eine hinführende Leitung oder Leitungsast 576, 577 und 578 als Abzweigung vom hinführenden Leitungsast 571 mit dem Verteilerkessel 190 verbunden. Weiterhin führt eine gemeinsame Sammelleitung 572.2, in den jeder Teilabschnitt einspeist, in einen gemeinsamen rückführenden Leitungsast 573. Die Wärmetauscher innerhalb eines jeden dieser Teilabschnitte sind zueinander parallelgeschaltet, sofern zwei oder mehr Wärmetauscher umfasst sind.

Hierbei ist der Leitungsknoten, mit dem die erste (Ab-)Leitung 576 des ND-Verteilerkreislaufs 582 von dem zentralen Leitungsast 571 abzweigt zwischen dem ersten Verdichter 192 und dem zweiten Verdichter 193 angeordnet, der Leitungsknoten, mit dem die zweite (Ab-)Leitung 577 des ND-Verteilerkreislaufs 582 vom zentralen Leitungsast 571 abzweigt, ist zwischen dem zweiten Verdichter 193 und dem dritten Verdichter 194 angeordnet und die dritte (Ab-)Leitung 578, die als letzte Teilstück des zentralen Leitungsastes 571 darstellt und einen Teil des HD-Verteilerkreislaufs 580 bildet, schließt sich stromabwärts zum dritten Verdichter 194 an. Hierbei versorgt die erste (Ab-)Leitung 576 alleinig den Wärmetauscher 302, den ND-Teilabschnitt des Leitungsabschnitts 572, die zweite (Ab-)Leitung 577 die beiden parallel geschalteten Wärmetauscher 322, 328, den MD-Teilabschnitt des Leitungsabschnitts 572, und die dritte (Ab-)Leitung 578 (HD-Verteilerkreislaufs 580) die drei parallel geschalteten Wärmetauscher 342, 352, 365, den HD-Teilabschnitt des Leitungsabschnitts 572. Somit weisen die drei (Ab-)Leitungen 576, 577 und 578 jeweils unterschiedliche Druckniveaus und unterschiedliche Temperaturniveaus auf. Die Einspeisung von flüssigem Medium über die (Sumpf-)Leitung 574 bzw. über die Abzweigungen in den zentralen Leitungsast 571, erfolgt analog zur Ausführung der Figur 2, wobei die Leitungsknoten der drei (Ab-)Leitungen in Strömungsrichtung vor dem Leitungsknoten der jeweiligen Einleitung aus der (Sumpf-)Leitung 574 angeordnet sind.

Der in der Darstellung untere Teilabschnitt, in den die Wärmetauscher 342, 352, 362 eingebunden sind (HD-Verteilerkreislauf), ist über die Druckregeleinheit 585 in der Sammelleitung 572.2, und der mittlere Teilabschnitt (ND-Verteilerkreislauf), in den die Wärmetauscher 322, 328 eingebunden sind, ist über die Druckregeleinheit 586 in der Sammelleitung 572.2 auf einem eigenständigen Druckniveau gehalten. In der Sammelleitung 572.2 erfolgt somit eine gestufte Entspannung.

Die drei Teilabschnitte des Leitungsabschnitts 572, über den die Energieverteilung an als Wärmesenken arbeitende Wärmetauscher erfolgt, münden in eine gemeinsame (Rück-)Leitung 573 und in den Verteilerkessel 190.

Vorteil dieser Ausführungsvariante gegenüber der aus Figur 2, ist der deutlich geringere Energiebedarf für den zweiten und dritten Verdichter 193, 194.

Der wasserführende Verteilerkreislauf weist am hinführenden Leitungsast 571 unmittelbar stromabwärts zum Verteilerkessel 190 ebenfalls eine Temperatur von ca. 133 °C und 3 bar auf. Nach dem ersten Verdichter 192 beträgt der Druck ca. 6 bar, der mit einem Energieverbrauch von ebenfalls 71 kW erreicht wird, nach dem zweiten Verdichter 194 beträgt der Druck ebenfalls ca. 12 bar, der ebenfalls mit einem elektrischen Energieverbrauch von 80 kW erreicht wird und nach dem dritten Verdichter 194 beträgt analogerweise der Druck 20 bar bei einer Temperatur von 250 °C, allerdings erreicht durch elektrischen Energieverbrauch von nur noch 42 kW für den dritten Verdichter 194. Dieser Vorteil stellt sich ein, weil der Volumenstrom im zweiten und dritten Verdichter 193, 194 stark verringert ist. Neben dem energetischen Vorteil ist hiermit verbunden, dass der dritte Verdichter 194 baulich wesentlich kleiner ausgelegt werden kann, was weiterhin in der Regel die Wartung und den Betrieb bzw. die Aufstellung ebenfalls verbessert. Ein weiterer Vorteil der Ausführungsform gem. Figure 3 ist, dass durch die gestufte Verdichtung und Abzweigung von einem Teilkreislauf des ND-Verteilerkreislaufs 582 im Verteilerkreislauf 570, im Zwischenkreislauf 560 zur Erreichung des Temperaturniveaus für die als Wärmesenke arbeitenden Wärmetauscher, nur eine Gebläsegruppe 176 mit drei Gebläsen 176.1, 176.2, 176.3 vorgesehen werden muss. Vorliegend kann der ND-Verteilkreislauf 582 auch als aus zwei Teilkreisläufen mit bestehend angesehen werden.

Zur Versorgung der in der Figur 3 gezeigten Wärmetauscher, die insb. die Sumpfwärmetauscher der Trennkolonnen sind, muss ein sehr hohes Temperaturniveau bereitgestellt werden, so dass über den Bedarfs- und Regelwärmetauscher 199 das zirkulierende Medium wieder gekühlt werden muss.

Grundsätzlich könnten in den ND-Teilabschnitt, in den der Wärmetauscher 302 eingebunden ist, auch die Wärmetauscher 206 der Reaktionseinheit 102 und/oder der Wärmetauscher 158 der Konditionierungseinheit 104 eingebunden werden.

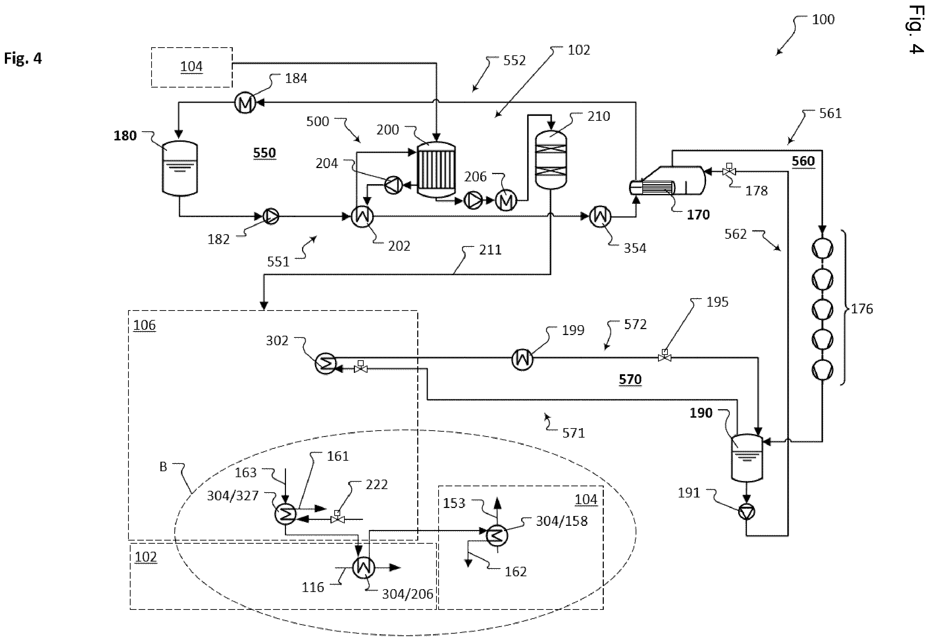

Im gezeigten Beispiel der Figur 3 sind die Wärmetauscher 304 (Kondensator) als Wärmequelle mit den als Wärmesenke arbeitenden Wärmetauscher 158, 206 und 327 über Medienleitungen direkt gekoppelt, d.h. seriell verschaltet. Hierbei sind als Wärmesenken der Wärmetauscher 327 in der (Feed-)Leitung 161 zur ersten Trennkolonne 320, der Wärmetauscher 206 im Vorlauf des Nachreaktors 210und der Wärmetauscher 158 in die (Feed-)Leitung 153 stromaufwärts zum Hauptreaktor 200 eingebunden. Diese Option einer direkten Wärmekopplung über Medienleitungen ist in dem unteren mit A gekennzeichneten Kasten in der Figur 3 dargestellt. Diese selektive Direktverschaltung zur seriellen EK mittels Medienleitungen und Verteilung der Wärme des Kondensators 304, hat gegenüber der Einbindung der Wärmesenken in den Verteilerkreislauf 570 gem. bspw. Fig. 2, wie vorstehend beschrieben, eine Effizienzsteigerung von 30 bis 50 % zur Folge. Der Vorteil stellt sich insb. ein, wenn mind. eine EK eine integrierte stoffbasierte EK ist, indem Wärmetauscher 304 mit dem (Vorlauf-)Wärmetauscher 158 und/oder dem (Feed-)Wärmetauscher 327 (nicht dargestellt; analog Fig. 4) stromaufwärts zur ersten Trennkolonne 320 der ersten Trennstufe 106B gekoppelt wird.

Das Ausführungsbeispiel, wie in der Figur 4 gezeigt ist, kann baulich einfache Lösung angesehen werden, mit der bereits 60 bis 80% der Energieeinsparung der Anlage erreicht werden, wie dies mit den vorherigen Lösungen der vollständigen oder weitgehend vollständigen Energieverschaltung erreicht wird. Hierbei umfasst der Verteilerkreislauf 570 nur einen Kreislauf. Dieser Verteilerkreislauf ist ohne Einbindung eines Verdichters im hinführenden Leitungsast 571. Das Druckniveau beträgt 3 bar und die Temperatur 133 °C.

Der Verteilerkessel 190 ist analog eingebunden in den Zwischenkreislauf 560, dessen Gebläsegruppe 176 fünf einzelne Gebläse 176.1 ... 176.5 aufweist.

Die Motivation für diese Ausführungsform kann darin bestehen, den Bedarf an elektrischer Leitung für den Betrieb von Verdichtern möglichst gering zu halten und trotzdem wesentliche Energieeinsparungen und insb. die Vermeidung von primären Energieformen zu erreichen, so dass nur der eine oder eine Gruppe von wenigen Wärmetauschern mit einem eher niedrigen Temperaturniveau, d.h. bei einer Temperatur von bis zu 130 bis 150 °C auf diesem Wegen gekoppelt und versorgt werden.

Im gezeigten Beispiel der Figur 4 ist eine weitere Option als Verbesserung oder Erweiterung gezeigt. Bei der ersten Option ist der Wärmetauscher 304 (Kondensator) analog zum Detailbild A der Figur 3 als Wärmequelle mit den Wärmetauschern 158, 206 und 327 verschaltet. Die Option einer Direktverschaltung von einzelnen Wärmetauschern ist in dem mit B gekennzeichneten Rahmen in der Figur 4 dargestellt. Im Unterschied zur Figur 3 und Rahmen A; erfolgt eine Direktverschaltung der Stoffströme der Leitungen 163/161, 163/116 und 163/153 zum indirekten Wärmeaustausch (serielle integrierte stoffbasierte EK) in jeweils einem Wärmetauscher 304/327, 304/206 und 304/158. Anders ausgedrückt, die erforderliche Kühlleistung in der Kopfleitung 163 wird in dem gezeigten Beispiel in den drei genannten Wärmetauschern erbracht und parallel die Stoffströme in den genannten Leitungen 161, 116, 153 erwärmt. Es kann in einer nicht gezeigten Ausführungsform vorgesehen werden, einen (Bedarfs-)Wärmetauscher vorzusehen, um die vollständige Kondensation in der (Kopf-)Leitung 163, 162 sicherzustellen.

Es hat sich überraschenderweise herausgestellt, dass insb. eine integrierte stoffbasierte EK durch die Integration der Wärmetauscher 304/327 in einem Bauteil und die Anordnung der Druckregeleinheit 222 stromaufwärts hierzu in der Leitung 161, zur wesentlichen Abkühlung des Stoffstroms in der Kopfleitung 163 führt und weiterhin den Energiebedarf des (Sumpf-)Wärmetauschers 322 der ersten Kolonne 320 wesentlich senkt. Durch die Druckregeleinheit 222 kann durch Entspannung im Zulauf 161 zum Wärmetauscher 304/327 das Temperaturniveau soweit abgesenkt werden, das eine Temperaturdifferenz von ca. 5 bis 10 °C zwischen der (Kopf-)Leitung 163 und der (Feed-)Leitung 161 vorliegt.

Diese Direktverschaltung der Stoffströme und Verteilung der Wärme des Kondensators 304, hat gegenüber der Einbindung der Wärmesenken in den Verteilerkreislauf 570 gem. Fig. 2, wie vorstehend beschrieben, eine Effizienzsteigerung von 30 bis 50 % zur Folge.

Vorliegend meint "Effizienzsteigerung", wenn nichts Abweichendes ausgeführt wird, einen geringeren Energieverbrauch. Der Bezug ergibt sich aus dem Zusammenhang und kann bezogen sein auf die Anlage, den jeweils beschriebenen Anlagenabschnitt oder den verbesserten Apparat bspw. durch Integration von zwei Wärmetauschern in einen einzigen.

Die zweite Option besteht in einer direkten Kopplung von zwei oder mehr Wärmetauschern, hier dem (Vor-)Wärmetauscher 327 der Trennkolonne 320 in der (Feed-)Leitung 161 mit dem (Vorlauf-)Wärmetauscher 206 des Nachreaktors 210 in der Leitung 116 (gestrichelt dargestellt).

Die Ausführungsform, in der nur der Wärmetauscher 302 im dem Verteilerkreislauf 570 vorgesehen wird, stellt ein besonders einfaches und effizientes Übertragungssystem dar, um einen erheblichen Teil des Wärmebedarfs mit hoher Effizienz zu decken. Allein durch diese Integration kann eine Effizienzsteigerung gegenüber einer Direktbeheizung der Wärmetauscher von ca. 40% erreicht werden. Die direkte Verschaltung der Wärmetauscher 304 als Wärmequelle mit den Wärmetauschern 206, 158 als (Verbraucher) Wärmesenken, führt zu einer Effizienzsteigerung von ca. 28%, d.h. Energieeinsparung von 28 %. Somit können durch beide Maßnahmen, den Verteilerkreislauf 570 und die Direktverschaltung des Kondensators 304 mit den Wärmetauschern 206, 158 eine Effizienzsteigerung von ca. 70 % erreicht werden.

Eine der beiden Optionen gem. den beiden Detailbildern A (Fig. 3), B (Fig. 4) können alternativ bei den Ausführungen nach Figuren 3 oder 4 vorgesehen werden.

Figur 5 zeigt eine verbesserte Variante der Reaktoreinheit 102. Hierbei sind zwei Hauptreaktoren 200, 201 schaltbar zueinander in Reihe geschaltet. Die steuer- und/oder regelbaren Ventile/- einheiten sind zum Teil in der Figur 5 eingezeichnet, weitere können vom Fachmann bedarfsweise vorgehen werden, um den sicheren Betrieb der beiden Hauptreaktoren 200, 201 sicherzustellen. Die beiden Hauptreaktoren 200, 201 sind jeweils in einen Kühlkreislauf 500, 501 eingebunden, mit dem jeweils der Festbettreaktor 200, 201 auf einer zulässigen, gekühlten Reaktionstemperatur gehalten wird. In der darstellten und mit vollausgezogenen Linien dargestellten Linien dargestellten Schaltung der Hauptreaktoren 200, 201 wird der erste Hauptreaktor 200 über die Leitung 110 kommend vom Mischbehälter 154 zuerst angeströmt und der erste Reaktionsanteil erfolgt in diesem Hauptreaktor 200. Stromabwärts über einen unteren Auslass und die Leitung 112 wird das Stoffgemisch in den Kopf des zweiten Hauptreaktors 201 geleitet und verlässt diesen über einen Bodenauslass und Leitung 115 in die gemeinsame Leitung 116 als Feed in den gemeinsamen Nachreaktor 210. In die gemeinsame Leitung 116 ist eine Wärmetauscher 206 eingebunden, worüber das für den Nachreaktor 210 erforderliche Temperaturniveau sichergestellt werden kann. Die Reihenfolge der Durchströmung der beiden Hauptreaktoren kann auch in umgekehrter Reihenfolge vom Hauptreaktor 201 zum Hauptreaktor 200 erfolgen. Hierzu wird in analoger Weise der Hauptreaktor 201 über die Leitung 111 zuerst angeströmt, wobei die Leitung 110 zum Kopf des Hauptreaktors 200 verschlossen ist. Das Stoffgemisch verlässt diesen Festbettreaktor über einen Bodenauslass und die Leitung (Zwischen-)Leitung 113, die mit dem Kopf des Hauptreaktors 200 verbunden ist, wobei die Leitung 115 verschlossen ist. Schließlich verlässt das Stoffgemisch den Hauptreaktor 200 über einen Bodenauslass und Leitung 114, die analog in die gemeinsame Leitung 116 mündet und damit zum Nachreaktor 210 führt. Aus beiden Kühlkreisläufen 500, 501 zweigt jeweils eine Leitung 502, 503 ab, die über jeweils einen Behälter 212, 213 geführt ist, die als Druckausgleichsbehälter dienen, und weiter zu einem Kamin und/oder einer vollständigen Oxidationseinheit. Die Leitungen und Ventile/-einheiten sind derart vorhanden und ausgelegt, dass jeder der Hauptreaktoren 200, 201 alleinige betrieben werden kann und der Stoffstrom um den jeweils anderen Hauptreaktor vollständig vorbeigeleitet werden kann. Die Fließrichtung der beiden Kühlkreisläufe 500, 501 ist durch einen Pfeil symbolisiert, wobei die beide Kühlkreisläufe 500, 501 vorteilhafterweise identisch oder im Wesentlichen gleich ausgebildet sind, da die beiden Hauptreaktoren 200, 201 abwechselnd bzgl. der Strömungsrichtung des Edukt- bzw. des Stoffstroms als erster bzw. zweiter Hauptreaktor betrieben werden.

Die Kühlkreisläufe 500, 501 sind allerdings derart ausgelegt und regelbar, dass je nach verfahrenstechnischen Bedarfen, wie insb. Produktleitung und/oder Produktqualität, jeweils eigenständige Kühlleistungen oder Kühlaufgaben erfüllt werden. Dies betrifft insb. den Volumenstrom pro Zeiteinheit an Kühlmedium und/oder das Temperaturniveau bzw. die zulässige Erwärmung des jeweiligen Kühlmediums.

In der Figur 5 ist weiterhin eine optionale Leitung 117 als gestrichelte Linie (Bypass 2) dargestellt, womit der Nachreaktor 210 umgangen werden kann, wenn beispielsweise dieser gewartet werden muss und/oder die Katalysatorfüllung erneuert werden muss. In diesem Fall wird mind. der in Strömungsrichtung des Stoffstroms zweite Hauptreaktor derart temperiert, dass die vollständige Reaktion bei der gewünschten Produktqualität, insb. dem gewünschten Anteil an den jeweiligen Isomeren sichergestellt ist. Der Leitungsabzweig der Leitung 117 kann in Strömungsrichtung vor oder nach Wärmetauscher 206 vorgesehen werden, wobei es vorteilhaft ist, diesen stromaufwärts zu Wärmetauscher 206 vorzusehen, um diese ggf. auch bypassen zu können und nötige Wartungsarbeiten bei laufender Anlage vornehmen zu können.

Die Umgehung des Hauptreaktors 200 kann im Betrieb des rechts im Bild dargestellten Hauptreaktors 201 über die Leitungen 111, 115 und 116 erfolgen. Die Umgehung des Hauptreaktors 201 kann im Betrieb des links im Bild dargestellten Hauptreaktors 200 über die Leitungen 110, 114 und 116 erfolgen, so dass jeweils die (Kreuz-)Leitungen 112, 113 zwischen den beiden Hauptreaktoren 200, 201 nicht durchflossen werden.

In den Kühlumläufen 500, 501 sind weiterhin (optional) bei der dargestellten Anlagenvariante Wärmetauscher 208, 209 eingebunden. Dieser werden mit einem Heizmedium betrieben, insb. Dampf, und dienen im Anfahrschritt der Hauptreaktoren 200 der (Vor-)Temperierung auf Reaktionstemperatur der jeweiligen Hauptreaktoren 200, 201. Das Niveau dieser Vortemperierung liegt bei ca. 80 bis 100 °C, idealerweise 85 °C bis 95 °C. In dem gezeigten Anlagen- und Verfahrensbeispiel, wie bereits zur Figur 1 ausgeführt, ist es bei dem Ziel, ein möglichst niedriges trans/trans-Isomerenverhältnis von ca. 17 bis 23 Gew.% zur realisieren vorteilhaft, wenn die mit Katalysator neu befüllten Hauptreaktor 200, 201 auf eine Temperatur von ca. 90 °C mittels des jeweiligen Wärmetauschers 208, 209 vorgeheizt werden, bzw. der jeweilige neu befüllte Hauptreaktor 200, 201 entsprechend vorbeheizt wird.

Durch die Vorheizung auf typischerweise 85 bis 95 °C wird ermöglicht, dass die Reaktion bei dem vorliegenden Katalysator unmittelbar starten kann, ohne oder im Wesentlich ohne Recyclingströme des Stoffgemisches der Prozesses bis zum Erreichen der gewünschten Reaktionstemperatur.

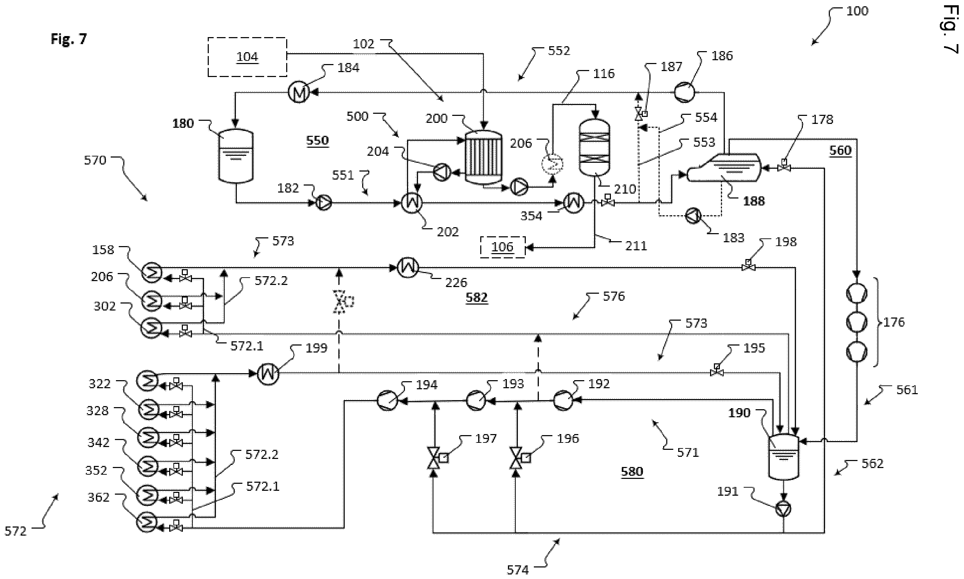

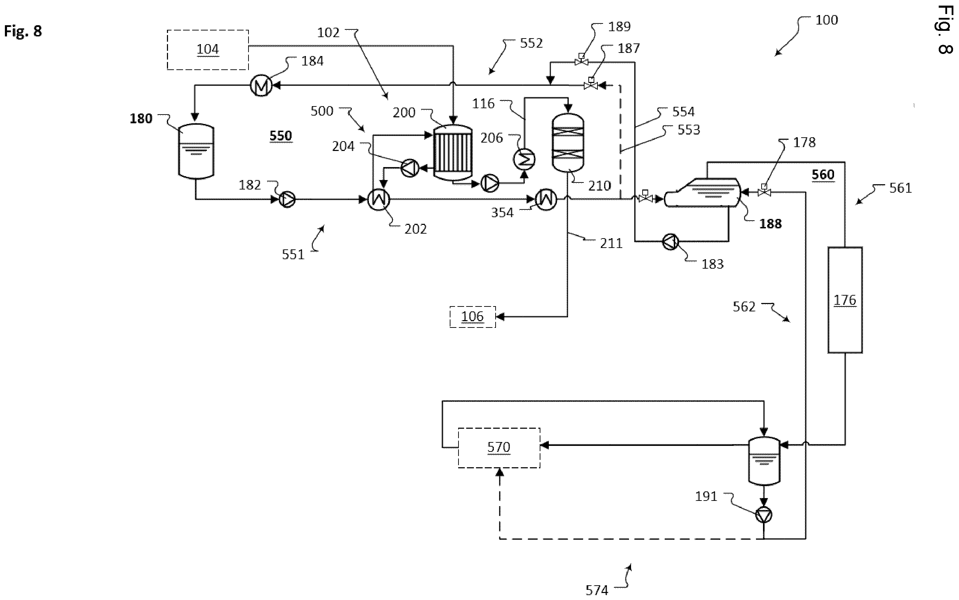

Die Variante der schaltbaren Hauptreaktoren 200, 201, wie sie in der Figur 8 dargestellt ist, unterscheidet sich zu der gem. Figur 7 darin, dass der Kühlkreislauf 500 des ersten Hauptreaktors 200 mit dem Kühlkreislauf des zweiten Hauptreaktors 201 ebenfalls in Reihe geschaltet ist. Hierbei ist nur ein (kühlender) Wärmetauscher 202 und nur eine Pumpe 204 für die gemeinsame Kühlung und den Medienumlauf vorgesehen, so dass der gemeinsame (zentrale) Leitungsast 508 in beiden Kühlkreisläufen 500, 501 in der Regel ständig und nur in eine Richtung durchströmt wird, unabhängig von der Verschaltung der beiden Hauptreaktoren 200, 201. Hierbei muss der "zentrale" Leitungsast natürlich nicht faktisch zwischen den beiden Hauptreaktoren 200, 201 angeordnet sein. Gezeigt ist die Variante als durchgezogenen Linien, bei der der erste Hauptreaktor 200 (links) zuerst mit dem Eduktgemisch über die Leitung 110 beschickt wird und auch die (Kühlmedien-)Einleitung nach dem Wärmetauscher 202 und der Pumpe 204 zuerst über diesen Hauptreaktor 200 erfolgt. Die in diese Schaltung nicht medienführenden Leitungen oder das Stoffgemisch führenden Leitungen sind gestrichelt dargestellt. Auch bei dieser Varianten besteht die Möglichkeit, das Stoffgemisch und/oder das (Kühl-)Medium vollständig an dem jeweils anderen Hauptreaktor vorbeizuleiten. Somit kann bspw. im Befüllvorgang eines der beiden Hauptreaktoren 200, 201, der jeweils andere unter maximaler Leistung weiter betrieben werden. Der Bypass des Stoffstroms bzw. des jeweiligen Hauptreaktors 200, 201 ergibt sich analog zu Figur 7.

In der gezeigten Beispielschaltung wird der zentrale Leitungsast 508 und der Wärmetauscher 202 durchflossen und über die Leitung 504 im Gleichstrom in den Medienraum ersten Hauptreaktors 200 geleitet, wobei zum gemeinsamen Leitungsknoten führende Leitung 505, kommend vom zweiten Hauptreaktor 201, gesperrt ist. Über die Leitung 506 verlässt das Medium an einem tiefen Auslass den ersten Hauptreaktor 202 und wird zu einem hohen Einlass in den Medienraum des zweiten Reaktors 201 geleitet (Kreuzleitung). Das Medium durchfließt den Medienraum des zweiten Hauptreaktors 201 ebenfalls im Gleichstrom und verlässt diesen an einem tiefliegenden Auslass über die Leitung 509, von der eine Abzweigung wieder in den zentralen Leitungsast 508 mündet, so dass der Kreislauf erneut durchflossen werden kann. Analog wird über die Leitung 505 der rechts dargestellte Hauptreaktor 201 zuerst durchströmt, wenn die Leitung 504 gesperrt ist. Das Medium verlässt dann den Medienraum des zweiten Hauptreaktors 201 über die Leitung 509 und wird an einem hochgelegenen Einlass in den Medienraum des anderen Hauptreaktors 200 geleitet. Der Auslass des Medienraums an einem tiefgelegenen Punkt führt in die Leitung 506 und von dort über eine Abzweigung in den zentralen Leitungsast 508.

Der besondere Vorteil des schaltbaren Hauptreaktors 200, 201 besteht in der Möglichkeit, den zuerst angeströmten Hauptreaktor bei einer höheren Temperatur zu betreiben, weil der Katalysator schon teilweise erschöpft und inaktiviert ist, im Wesentlichen ohne hierbei das trans/trans-Isomerenverhältnis negativ zu beeinflussen (d.h. zu erhöhen). Parallel hierzu fällt stärker erwärmtes Kühlmedium im jeweiligen (Umlauf-)Wärmetauscher 202, 203 des zuerst durchflossenen Hauptreaktors 200, 201 an. Dieses heißere Wärmetauschmedium kann aufgrund der höheren Temperatur besser in der Anlage zur integrierten medienbasierten EK und/oder zum üblichen Wärmetausch mit einem Stoff- Eduktstrom eingesetzt werden.

In der Figur 6 ist analoge zur Figur 5 die optionale Leitung 117 als gestrichelte Linie (Bypass 2) dargestellt, womit der Nachreaktor 210 umgangen werden kann, wobei die Leitung 117 stromabwärts zum (Vorlauf-)Wärmetauscher 206 des Nachreaktors 210 abzweigt.

In der Figur 6 ist weiterhin eine Anlagenvariante (gestrichelt) dargestellt, indem zur Erlangung einer höheren Sicherheit bzw. eines höheren Freiheitsgrades des Temperaturmanagements in der jeweiligen (Kreuz-)Leitung 504, 505 der Kühlkreisläufe 500, 501 bedarfsweise schaltbare und regelbare Wärmetauscher 203 (Vorlaufkühler) angeordnet sind. In dem gezeigten Ausführungsbeispiel sind die beiden Wärmetauscher 202 in Reihe geschaltet, da durch die jeweils inaktive (Kreuz-)Leitung, indem gezeigten Beispiel die Leitung 505, am Einbauort für den Wärmetauscher 202 keine Erwärmung erfolgt. Die Kühlmedienleitung bzw. der Kühlmedienkreislauf der Wärmetauscher 202 kann bei einer vorteilhaften Variante in einem Seitenstrom bzw. über einen Neben- oder Hilfskreislauf über die Pumpe 204 betrieben werden.

Der große Vorteil dieses Seitenstroms oder Neben-/Hilfskreislaufes eines Kühlmittels über die Wärmetauscher 203 ist, dass diese zusätzliche Kühlleistung nur bedarfsweise abgerufen werden braucht und mit geringem Aufwand eine weitergehend, bedarfsmäßige Regelung der Temperatur im jeweils zweiten beiden seriell geschalteten Hauptreaktoren erfolgen kann.

In der Figur 7 ist weiterhin eine Anlagenvariante dargestellt, die analog insb. zur Figur 2 ausgebildet ist. Im Unterschied zu den vorherigen Ausführungsformen und Varianten der Figuren 2 bis 4, ist der Sammelkreislauf 550 mit dem Zwischenkreislauf 560 ebenfalls über einen Verteilerkessel 188 gekoppelt. Der erwärmte Stoffstrom der hinführenden Leitung 551 wird also offen in den Innenraum des Verteilerkessels 188 entlassen, analog zum Verteilerkessel 190, so dass eine Erwärmung und Verdampfung im Verteilerkessels 188 über die Einleitung des Medienstroms, hier Heißwasser, aus der Leitung 551 über ein nicht näher bezeichnetes Entspannungsventil erfolgten kann. Weiterhin ist in der rückführenden Leitung 552 des Sammelkreislaufs 550 ein Verdichter vorgesehen, um einen definierten Dampfrücklauf zum Sammelkessel 180 sicherzustellen. Weiterhin sind als gestrichelte Linien Leitung 553, 554 dargestellt, als Optionen zur verbesserten Regelung des Füllstandes in dem Verteilerkessel 188. Die (Medien-)Leitung 553 ist eine Bypass-Leitung zum Verteilerkessel 188 und die (Sumpf-)Leitung 554, in die auch eine Pumpe 183 eingebunden ist, ein Entnahmeweg, um einen definierten Füllstand und hierdurch auch die Zulaufmenge an heißem Medium aus der hinführenden Leitung 551 zu regeln.

Bei der zur Figur 7 alternativen Ausführungsform der Figur 8, ist am Verteilerkessel 188 nur eine Kopfauslass oder Kopfleitung für den Anschluss der Leitung 561 vorgesehen, nicht für die rückführende Leitung 552 des Sammelkreislaufs 550. Die hinführende Leitung 551 hat einen analogen Anschluss an den Verteilerkessel 188, der über ein nicht näher bezeichnetes Entspannungsventil führt. Die (Sumpf-)Leitung ist bei dieser Ausführungsform stromabwärts zur Druckregeleinheit 187 in die rückführende Leitung 552 über einen Leitungsknoten eingebunden, zu Einstellung des erforderlichen Druckniveaus ist zusätzlich in der (Sumpf-)Leitung 554 eine Druckregeleinheit 189 vorgesehen. Im Regelbetrieb ist die (Bypass-)Leitung 553 nicht durchflossen. Auch hier beseht bedarfsweise die Option, den Füllstand und auch die Zulaufmenge im Verteilerkessel 188 gezielt zu regeln. Ein weiterer Vorteil dieser Lösung ist, dass die im fluiden Medium arbeitende (Sumpf-)Pumpe 183, mit geringerem Energieverbrauch gegen die Saugleitung der Gebläsegruppe 176 arbeiten kann, also dies für den Verdichter 186 gem. Figur 7 erforderlich ist.

Der (Vorlauf-)Wärmetauscher 206 ist vorliegend vorrangig beschrieben und teilweise in einer EK gezeigt, bei denen dieser als Wärmetauscher 206 vorrangig als Wärmesenke arbeitet, also der darin geführte Stoffstrom erwärmt wird. Durch die an den Hauptreaktor 200, 201 angepasste, abhängige Betriebsweise des Nachreaktors 210 kann mind. zeitweise, insb. dauerhaft, eine Kühlung des Stoffstroms in der (Feed-)Leitung 116 stromaufwärts zum Nachreaktor 210 erforderlich sein, weil im (adiabatischen) Nachreaktor ca. 10 bis 20% des Umsatzes erfolgen, so dass eine Erwärmung des Stoffstroms gemessen im Auslass bis auf ca. 140 °C erfolgt. Somit kann unabhängig von den hierhin beschriebenen Ausführungsformen und Varianten der Anlagen und des Verfahrens eine angepasste EK zur Kühlung (Wärmetauscher 206 arbeitet als Wärmequelle) vorgesehen werden. Alternativ oder zusätzlich kann eine ergänzende Kühlung über eine geänderte oder weitere EK vorgesehen werden.

Insgesamt sind eine Vielzahl von üblichen, dem Fachmann bekannte und für eine vorteilhafte Prozessführung nötige oder sinnvolle Steuer- und Regelungselemente nicht dargestellt, wie Sensoren (Durchfluss, Temperatur, Druck etc.), Anzeigen, Stell- und Regelglieder (insb. Ventile, weitere Pumpen, Verdichter), Sammelbehälter etc. und sind bedarfsweise zu ergänzen. Insb. sind auch bei der Nennung von "einer" Pumpe" oder "einem" Verdichter übliche Redundanzen aus mind. zwei parallelen Aggregaten gemeint, insb. von zwei parallelen Pumpen oder zwei parallelen Verdichtern. In analoger Weise ist "ein Wärmetauscher" nicht limitierend zu verstehen und meint mit der jeweiligen, örtlichen Wärmetauschaufgabe auch Anordnungen von in Reihe geschalteten Wärmetauschern oder parallel geschaltete, auch redundanten, Wärmetauschern, wobei hiermit keine Verschaltungen mit mind. einem weiteren Wärmetauscher und örtlich unterschiedlicher Wärmetauschaufgabe zu verstehen ist.

Grundsätzlich sind alle Wärmetauscher und Kondensatoren derart ausgebildet, dass ein indirekter Wärmeübertrag erfolgt und keine stoffliche Vermischung von Edukten, Produkt, Nebenprodukten und/oder Lösungsmittel mit den (Heiz-/Kühl-)Medium, wie Gas, Dampf, Wasser, Öl, Sole (Brine) etc. erfolgt, es sei denn es ist etwas hiervon Abweichendes ausgeführt.

Auch wenn vorliegend Bauteile, wie Ventile, Druckregeleinheit, Absperraggregate etc. zur vereinfachten Beschreibung einzeln oder gesondert dargestellt sind und zum Teil nicht einzeln genannt wurde, ist dies nicht limitierend zu lesen, vielmehr kann der Fachmann bedarfsweise zwei oder mehrere dieser Bauteile in einer Ventil- oder Stelleinheit zusammenfassen oder Mehrwegventile stattdessen vorsehen.

Vorliegend meint "stromaufwärts" oder "stromabwärts" die Anordnung und/oder Fließrichtung des produktreichen Stoffstroms, wenn nichts hiervon Abweichendes ausgeführt ist. Weiterhin ist unter "Medien", "Medienstrom", "Medienleitung" etc. immer ein Heiz- oder Kühlmedium bzw. die zugehörige Leitung gemeint, wenn nichts hiervon Abweichendes ausgeführt ist.

Die Begriffe "Sumpfablauf", "Sumpfauslauf", "Sumpfableitung" oder "Sumpfausleitung" werden zum Teil synonym verwendet, ebenso werden in analoger Weise die Begriffe "Kopfablauf, - auslauf, - ableitung oder -ausleitung" zum Teil synonym verwendet.

Weiterhin ist der Begriff "Wärmetauscher, der als Kondensator betrieben wird" breit auszulegen, und meint auch eine unvollständige Kondensation oder Abkühlung des zugeleiteten Stoffstroms, so dass ein "Wärmetauscher, der als Kondensator betrieben wird", auch teilweise synonym als "Kondensator" benannt wird.

Der Begriff "Sumpfpumpe" meint eine in einen Sumpfumlauf eines Apparates (Trennkolonne, Behälter, etc.) eingebundene Pumpe und/oder eine sich stromabwärts zum Sumpfauslass eines Apparates befindliche Pumpe, die zur Förderung flüssigen Stoffstroms vorgesehen ist.

Für alle Ausführungsformen und Varianten der Anlage und des Verfahrens kann generell vorgesehen werden, dass die zweite Trennkolonne 340 und die dritte Trennkolonne 350 als eine einzige Kolonne ausgeführt werden, insb. als eine Trennwandkolonne (nicht dargestellt). Hierbei können vorteilhafterweise die Trennaufgaben der zweite Trennkolonne 340 und der dritten Trennkolonne 350 mind. teilweise, idealerweise vollständig mittels der Trennwandkolonne (nicht dargestellt) ausgeführt werden, wie sie bspw. aus den Druckschriften EP 012 62 88 B1 oder EP 012 23 67 A2 bekannt sind.

Insgesamt kann ein großer, energetischer Vorteil mit der erfindungsgemäßen Anlage und dem Verfahren erreicht werden, der darin besteht, dass eine starke Verringerung der extern zugeführten Energieströme ermöglicht wurde, insb. die Einsparung von großen Mengen an (externem) Heizdampf.

## Patentansprüche

1. Anlage (100) zur kontinuierlichen, katalytischen Hydrierung von Methylendianilin (MDA; Edukt1) mit einem Wasserstoffgeber (Edukt2), insb. einem gasförmigen Wasserstoffgeber, vorzugsweises Wasserstoff (H2),
umfassend eine Konditionierungseinheit (104) für die Edukte, eine Reaktoreinheit (102) und eine Trenneinheit (106), wobei
- die Konditionierungseinheit (104) (Zu-)Leitungen für Edukte1, Edukt2 und mind. ein Lösungsmittel, mind. einen Wärmetauscher (158) in mind. einer (Zu-)Leitung, mind.
einen Mischer (152) zur Mischung der Edukte und/oder mind. eines Edukts mit mind.
einem Lösungsmittel umfasst;
- die Reaktoreinheit (102) mind. einen Festbettreaktor als Hauptreaktor (200, 201) mit einer immobilen Katalysatorpackung umfasst, wobei der mind. eine (erste) Hauptreaktor (200, 201)
- einen ersten Strömungsweg für das Stoffgemisch
über die immobile Katalysatorpackung und
- einen weiteren hiervon getrennten, geschlossenen Strömungsweg für ein Wärmetauschmedium außerhalb der Katalysatorpackung umfasst, und
wobei in den Medienumlauf ein Wärmetauscher (202) eingebunden ist;
- die Trenneinheit (106) mind.
- eine erste Trennstufe (106A) zur (im Wesentlichen) Abtrennung des Lösungsmittels
und
- eine zweite Trennstufe (106B) zur Abtrennung vom mind. einem Edukt und/oder mind. einem Nebenprodukt vom Produkt umfasst,
**dadurch gekennzeichnet, dass**
i) ein Sammelkreislauf (550) für einen offenen oder einen geschlossenen ersten Medienumlauf umfasst ist, in den als mind. eine Wärmequelle ein Wärmetauscher (202, 206) der Reaktoreinheit (102), insb. der in den Medienumlauf (500) des Reaktors (200) eingebundene Wärmetauscher (202), und/oder mind. ein Wärmetauscher (354) der Trenneinheit (106) sowie ein Verdampfer (170) oder ein Verteilerkessel (188) eingebunden sind,
ii) ein Zwischenkreislauf (560) für einen zweiten Medienumlauf umfasst ist, in den der erste Verdampfer (170), mind. ein Gebläse (176.1) und ein Verteilerkessel (190) eingebunden sind, wobei von der Druckseite des mind. einen Verdichters (176.1) eine (Medien-)Leitung zum Verteilerkessels (190) führt und ein (Sumpf-)Auslass des Verteilerkessels (190) über eine rückführende Leitung (562) mit einem Einlass des Verdampfers (170) verbunden ist, und wobei
iii) mind. ein Verteilerkreislauf (570) umfasst ist, in den der Verteilerkessels (190) eingebunden ist, wobei der Verteilerkreislauf (570) mit mind. einem hinführenden Leitungsast (571) an einen Kopf-/Dampfauslass des Verteilerkessels (190) angeschlossen ist, wobei der Verteilerkreislauf (570) mind. einen verteilenden Leitungsast (572) und mind. einen rückführenden Leitungsast (573) umfasst, wobei in den mind. einen verteilenden Leitungsast (572) als Wärmesenke mind. ein Wärmetauscher (158, 206, 302, 322, 328, 342, 352, 362) der Konditionierungseinheit (104), der Trenneinheit (106), und/oder der Reaktionseinheit (102) wärmetauschend eingebunden sind, und wobei
der Zwischenkreislauf (560) und der Verteilerkreislauf (570) über den Verteilerkessel (190) medienleitend miteinander verbunden sind.

2. Anlage nach Anspruch 1, **dadurch gekennzeichnet, dass** in dem mind. einen hinführenden Leitungsast (571) des Verteilerkreislaufs (570) mind. ein Verdichter (192, 193) eingebunden ist.

3. Anlage nach Anspruch 1 oder 2, **dadurch gekennzeichnet, dass** der Verteilerkreislauf (570) mind. die zwei folgenden (Teil-)Kreisläufe umfasst,
- mind. einen Hochdruck-Verteilerkreislauf (HD-Verteilerkreislauf) (580), der an einen Kopf-/Dampfauslass des Verteilerkessels (190) angeschlossen ist und in den mind. ein Verdichter (192, 193) eingebunden ist und
- mind. einen Niederdruck-Verteilerkreislauf (ND-Verteilerkreislauf) (582), der an einen Kopf-/Dampfauslass des Verteilerkessels (190) angeschlossen ist und in den kein Verdichter, weniger Verdichter und/oder eine geringere Verdichterleistung eingebunden ist, als in den HD-Verteilerkreislauf, und wobei in den HD-Verteilerkreislauf (580) und den ND-Verteilerkreislauf (582)
- als Wärmesenke mind. ein Wärmetauscher (158, 206, 302, 322, 328, 342, 352, 362) der Konditionierungseinheit (104), Trenneinheit (106), und/oder der Reaktionseinheit (102) wärmetauschend eingebunden sind, und wobei der Zwischenkreislauf (560) und der Verteilerkreislauf (570) über den Verteilerkessel (190) medienleitend miteinander verbunden sind.

4. Anlage nach einem der vorherigen Ansprüche, **dadurch gekennzeichnet, dass** im Zwischenkreislauf (560) mind. einen Gebläse (175.1) oder eine Gruppe von x Gebläsen (175.1, 175.2 ... 175.x) umfasst, wobei x >= 2 ist, insb. >= 3.

5. Anlage nach einem der vorherigen Ansprüche, **dadurch gekennzeichnet, dass** in den Medienumlauf des Sammelkreislaufs (550) ein Sammeltank (180) und eine Pumpe (182) eingebunden sind, wobei ein Wärmetauscher (182) im rückführenden Leitungsast (552) stromaufwärts zum Sammeltank (180) und/oder im hinführenden Leitungsast (551) auf der Saugseite der Pumpe (182) angeordnet ist.

6. Anlage nach einem Ansprüche 3 bis 5, **dadurch gekennzeichnet, dass** der HD-Verteilerkreislauf (580) des Verteilerkreislaufes (570) einen ersten Leitungsast (571) als dampfführende Zuleitung umfasst, wobei in den zuführenden Leitungsast (571) mind. ein Verdichter (192, 193, 194) eingebunden ist.

7. Anlage nach einem der Ansprüche 2 bis 6, **dadurch gekennzeichnet, dass** vom Verteilerkessel (190) eine (Sumpf-)Leitung (574) auf die Druckseite des mind. einen Verdichters (192) führt, wobei in diese (Sumpf-)Leitung (574) eine Pumpe (191) eingebunden ist.

8. Anlage nach Anspruch 7, **dadurch gekennzeichnet, dass** die Sumpfleitung (574) vom Verteilerkessel (190) in die (Medien-)Leitung (571) zwischen zwei Verdichtern (192, 194) geführt ist.

9. Anlage nach einem der Ansprüche 7 oder 8, **dadurch gekennzeichnet, dass** die Sumpfleitung (574) mind. zwei (Leitungs-)Äste aufweist, wobei jeder (Leitungs-)Ast der der Sumpfleitung (574) zwischen zwei der Verdichter (192, 194, 194) geführt ist, und wobei mind. ein (Leitungs-)Ast der Sumpfleitung (574) eine Druckregeleinheit(196, 197) umfasst, insb. jeder (Leitungs-)Ast eine Druckregeleinheit(196, 197) umfasst.

10. Anlage nach einem der vorherigen Ansprüche, **dadurch gekennzeichnet, dass** ND-Verteilerkreislauf (582) des Verteilerkreislaufs (570) mind. zwei Teilkreisläufe zur Verteilung- oder Abgabe von Energie umfasst, wobei
- der erste Teilkreislauf ein Niederdruckkreislauf (ND-Teilkreislauf) ist, wobei in den ersten ND-Teilkreislauf mind. ein Wärmetauscher (302) oder eine erste Gruppe aus zwei oder mehr Wärmetauschern eingebunden ist, und wobei
- in den weiteren Teilkreislauf mind. ein Wärmetauscher (322, 328) oder eine weitere Gruppe aus zwei oder mehr Wärmetauschern eingebunden ist, wobei in den ND-Teilkreislauf kein Verdichter, weniger Verdichter und/oder eine geringere Verdichterleistung eingebunden ist, als in jeden weiteren Teilkreislauf den ND-Verteilerkreislaufs (580).

11. Anlage nach Anspruch 10, **dadurch gekennzeichnet, dass** in mind. eine der rückführenden Leitungen (573, 575), insb. in jede zum Verteilerkessel (190) rückführende Leitung (572.2, 573) des Verteilerkreislaufs (570) ein Wärmetauscher (199, 226) eingebunden ist.

12. Anlage nach einem der vorherigen Ansprüche, **dadurch gekennzeichnet, dass** die als Wärmesenke wirkenden Wärmetauscher (158, 206, 302, 322, 328, 342, 352, 362) des Verteilerkreislaufs (570) parallel geschaltet sind und/oder die jeweilige Gruppen von zwei oder mehr Wärmetauschern (158, 206, 302, 322, 328, 342, 352, 362) des HD-Verteilerkreislaufs (580) und/oder des ND-Verteilerkreislaufs (582) zueinander parallel geschaltet sind.

13. Anlage nach einem der vorherigen Ansprüche, **dadurch gekennzeichnet, dass** die als Wärmequelle wirkenden Wärmetauscher (202, 354) des Sammelkreislaufs (550) in Reihe geschaltet sind.

14. Verfahren zur katalytischen Hydrolyse von Methylendianilin (MDA; Edukt1) mit einem
Wasserstoffgeber (Edukt2), insb. einem gasförmigen Wasserstoffgeber, vorzugsweises Wasserstoff (H2),
wobei die Herstellung mittels einer industriellen Anlage (100) erfolgt,
**dadurch gekennzeichnet, dass**
die Anlage (100) nach mindestens einem der vorstehenden Vorrichtungsansprüche ausgebildet ist, wobei der Hauptreaktor (200) bei einer Temperatur im Bereich von 80 °C bis 150 °C betrieben wird, und wobei durch Dampfkompression im Zwischenkreislauf (560) mittels des mind. einen Gebläses (176.1) und/oder der Gebläsegruppe (176) mind. eine Temperaturerhöhung des Mediums in dem hinführenden Leitungsast (561) um 20 °C bis 60 °C vorgenommen wird, idealerweise von 30 °C bis 50 °C.

15. Verfahren nach Anspruch 14, **dadurch gekennzeichnet, dass** die Temperatur des Eduktstroms am Einlass des mind. einen Hauptreaktors (200, 201) 80 bis 135°C beträgt, idealerweise 90 bis 120 °C.

16. Verfahren nach einem der vorherigen Verfahrensansprüche, **dadurch gekennzeichnet,**
**dass** der Druck im Hauptreaktor (200, 201) im Bereich von 60 bar bis 120 bar betrieben liegt, idealerweise im Bereich von 70 bis 110 bar.

17. Verfahren nach einem der vorherigen Verfahrensansprüche, **dadurch gekennzeichnet,**
**dass** dieses kontinuierlich, katalytisch zur Produktion von Methylenbis(cyclohexylamin) erfolgt, insb. zur Produktion von 4,4'-Diaminodicyclohexylmethan (PACM), bevorzugt 4,4`-Diaminodicyclohexylmethan (PACM) mit niedrigen Anteilen an trans/trans-Isomeren.

18. Verfahren nach einem der vorherigen Verfahrensansprüche, **dadurch gekennzeichnet,**
**dass** neben dem mind. einen Hauptreaktor (200, 201) weiterhin mind. ein Katalysator enthaltender Nachreaktor (210) umfasst ist, wobei der mind. eine Hauptreaktor (200, 201) und der mind. eine Nachreaktor (210) bei demselben oder im Wesentlichen bei demselben Druck betrieben werden.

19. Verfahren nach einem der vorherigen Verfahrensansprüche, **dadurch gekennzeichnet, dass** die vom Sammelkreislauf (550) in den ersten Verdampfer (170) eingebrachte Energie mittels des Zwischenkreislaufs (560) und dem mind. einen eingebundenen Gebläse (176.1) und/oder der Gruppe (176) aus x Gebläsen (176.1, 176.2 ... 176.x)
- um mind. Faktor 1,1 bis 2,5 und/oder
- die Ausgangstemperatur des Kessels (190) um xx °C bis °C angehoben wird, idealerweise um aa °C bis bb °C.
angehoben wird.

20. Verfahren nach einem der vorherigen Verfahrensansprüche, **dadurch gekennzeichnet, dass** im HD-Verteilerkreislauf (580) im ersten Leitungsteil (A-Teil) eine mehrstufige Druckerhöhung in der hinführenden Leitung (573) erfolgt, wobei nach dem Verteilerkessel (190) und
- vor dem ersten Verdichter (192) in der Leitung (571) ein (Eingangs-)Druck von 1,5 bar bis 5 bar, idealerweise 2 bar bis 4 bar und eine Temperatur von 100°C bis 150°C, idealerweise 120 bis 145°C vorliegen und/oder
- nach dem letzten Verdichter (194) und vor dem ersten als Wärmesenke wirkenden Wärmetauscher (662) ) in der Leitung (571) ein (End-)Druck von 10 bis 30 bar, idealerweise 15 bis 25 bar, und eine Temperatur von 180°C bis 300°C, idealerweise von 220 bis 280°C, vorliegen.
